# EUROPÄISCHE PATENTSCHRIFT

(11) **EP 2 595 955 B1**
(45) Veröffentlichungstag und Bekanntmachung des Hinweises auf die Patenterteilung: **30.11.2016**
(21) Anmeldenummer: 11734081.0
(22) Anmeldetag: 19.07.2011
(51) Int. Cl.: C07C 317/24, C07C 317/44, C07C 323/22, C07C 323/62, C07C 323/65, C07D 493/08, A01N 35/06, A01N 37/40, A01N 41/10, A01N 43/12

(54) **(4-HALOGENALKYL-3-THIOBENZOYL)CYCLOHEXANDIONE UND IHRE VERWENDUNG ALS HERBIZIDE**
(4-HALOGENALKYL-3-THIOBENZOYL)CYCLOHEXANEDIONES AND USE THEREOF AS HERBICIDES
(4-HALOGÉNOALKYLE-3-THIOBENZOYLE)CYCLOHEXANEDIONES ET LEUR UTILISATION COMME HERBICIDES

(30) Priorität: 21.07.2010 EP 10170231
(43) Veröffentlichungstag der Anmeldung: 29.05.2013
(73) Patentinhaber: Bayer Intellectual Property GmbH, 40789 Monheim am Rhein (DE)
(72) Erfinder: AHRENS, Hartmut, 63329 Egelsbach (DE); FEUCHT, Dieter, 65760 Eschborn (DE); GATZWEILER, Elmar, 61231 Bad Nauheim (DE); HÄUSER-HAHN, Isolde, 51375 Leverkusen (DE); ROSINGER, Christopher, Hugh, 65719 Hofheim (DE)
(74) Vertreter: BIP Patents
(86) Internationale Anmeldenummer: PCT/EP2011/062306
(87) Internationale Veröffentlichungsnummer: WO 2012/010575

(56) Entgegenhaltungen:
- EP-A2- 0 338 992
- EP-B1- 2 595 963
- WO-A1-2012/010574
- DE-A1- 19 961 465
- US-A1- 2002 165 096

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bereits bekannt, daß bestimmte Benzoylcyclohexandione herbizide Eigenschaften besitzen. So werden in EP 0 338 992 A1 und in WO 2011/012247 A1 jeweils 2-(3-Alkylthiobenzoyl)cyclohexandione genannt, die durch verschiedene Reste substituiert sind. US 2002/165096 A1 und DE 19961465 A1 offenbaren Benzoylcyclohexandione, die in 3-Position des Phenylrings einen direkt gebundenen Schwefel-Rest tragen.

Die aus diesen Schriften bekannten Verbindungen zeigen jedoch häufig eine nicht ausreichende herbizide Wirksamkeit. Aufgabe der vorliegenden Erfindung ist daher die Bereitstellung von herbizid wirksamen Verbindungen mit - gegenüber den aus dem Stand der Technik bekannten Verbindungen - verbesserten herbiziden Eigenschaften.

Es wurde nun gefunden, daß bestimmte Benzoylcyclohexandione, deren Cyclohexandion-Ring überbrückt ist, und deren Phenylring in 3-Position eine Thiogruppe und in 4-Position eine Halogenalkylgruppe trägt, als Herbizide besonders gut geeignet sind. Ein Gegenstand der vorliegenden Erfindung sind (4-Halogenalkyl-3-thiobenzoyl)cyclohexandione der Formel (I) oder deren Salze 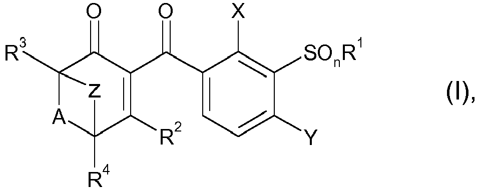 worin
X bedeutet (C₁-C₄)-Alkyl,
Y bedeutet (C₁-C₄)-Halogenalkyl mit Ausnahme von Trifluormethyl,
A und Z bedeuten unabhängig voneinander Sauerstoff, -S(O)ₘ-, -N(R⁵)-, Carbonyl oder (C₁-C₄)-Alkylen, das durch q Einheiten aus der Gruppe Sauerstoff, -S(O)ₘ-, -N(R⁵)- und Carbonyl unterbrochen ist, und das durch s Reste R⁶ substituiert ist,
R¹ bedeutet (C₁-C₄)-Alkyl,
R² bedeutet Hydroxy, SR⁷, NR⁸R⁹,
R³ und R⁴ bedeuten unabhängig voneinander Wasserstoff oder (C₁-C₄)-Alkyl,
R⁵ bedeutet Wasserstoff, (C₁-C₄)-Alkyl, (C₁-C₄)-Alkoxy, (C₁-C₄)-Alkylcarbonyl, (C₁-C₄)-Alkoxycarbonyl, Phenylcarbonyl oder Phenoxycarbonyl, wobei der Phenylring in den beiden letztgenannten Resten durch bis zu p Halogenatome und durch bis zu p Reste aus der Gruppe Nitro, Cyano, (C₁-C₄)-Alkyl, (C₁-C₄)-Halogenalkyl, (C₁-C₄)-Alkoxy und (C₁-C₄)-Halogenalkoxy substituiert ist,
R⁶ bedeutet Halogen, (C₁-C₄)-Alkyl, (C₁-C₄)-Halogenalkyl, (C₁-C₄)-Alkoxy, (C₁-C₄)-Halogenalkoxy oder (C₁-C₄)-Alkoxy-(C₁-C₄)-alkyl,
R⁷ bedeutet (C₁-C₄)-Alkyl oder durch p Halogenatome oder durch p Reste aus der Gruppe Nitro, Cyano, (C₁-C₄)-Alkyl, (C₁-C₄)-Halogenalkyl, (C₁-C₄)-Alkoxy und (C₁-C₄)-Halogenalkoxy substituiertes Phenyl,
R⁸ bedeutet Wasserstoff, (C₁-C₄)-Alkyl oder (C₁-C₄)-Alkoxy,
R⁹ bedeutet Wasserstoff oder (C₁-C₄)-Alkyl,
   oder
R⁸ und R⁹ bilden mit dem Stickstoffatom, an das sie gebunden sind, einen 5- oder 6-gliedrigen gesättigten, partiell gesättigten oder vollständig ungesättigten Ring, der m weitere Heteroatome aus der Gruppe Sauerstoff, Schwefel und Stickstoff enthält, und der durch bis zu s Halogenatome und durch bis zu p Reste aus der Gruppe, Cyano, (C₁-C₄)-Alkyl, (C₁-C₄)-Halogenalkyl, (C₁-C₄)-Alkoxy und (C₁-C₄)-Halogen-alkoxy substituiert ist,
m und n bedeuten unabhängig voneinander 0, 1 oder 2,
p bedeutet 0, 1, 2, 3, 4 oder 5,
q bedeutet 0 oder 1,
s bedeutet 0, 1, 2, 3, 4, 5, 6, 7 oder 8.

Für den Fall, daß R² Hydroxy bedeutet, können die erfindungsgemäßen Verbindungen der Formel (I) in Abhängigkeit von äußeren Bedingungen, wie Lösungsmittel und pH-Wert, in unterschiedlichen tautomeren Strukturen auftreten:

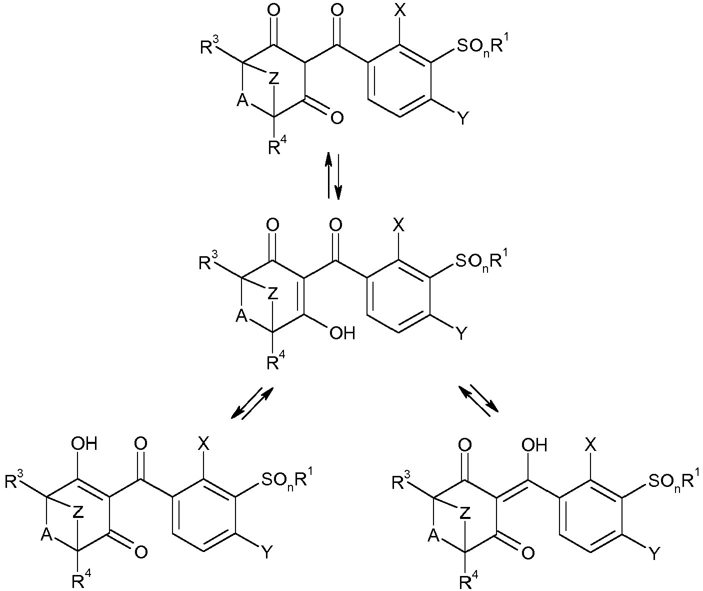

Je nach Art der Substituenten enthalten die Verbindungen der allgemeinen Formel (I) ein acides Proton, das durch Umsetzung mit einer Base entfernt werden kann. Als Basen eignen sich beispielsweise Hydride, Hydroxide und Carbonate von Lithium, Natrium, Kalium, Magnesium und Calcium sowie Ammoniak und organische Amine wie Triethylamin und Pyridin. Ebenso können Salze gebildet werden durch Anlagerung von organischen Säuren, wie Ameisen- oder Essigsäure, und anorganischen Säuren, wie Phosphor-, Salz- oder Schwefelsäure. Solche Salze sind ebenfalls Gegenstand der Erfindung.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder lod.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrisch substiuierte Kohlenstoffatome oder Sulfoxide vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Von besonderem Interesse sind Verbindungen der allgemeinen Formel (I), worin
X bedeutet (C₁-C₄)-Alkyl,
Y bedeutet (C₁-C₄)-Halogenalkyl mit Ausnahme von Trifluormethyl,
A und Z bedeuten unabhängig voneinander Sauerstoff oder (C₁-C₄)-Alkylen,
R¹ bedeutet (C₁-C₄)-Alkyl,
R² bedeutet Hydroxy,
R³ und R⁴ bedeuten unabhängig voneinander Wasserstoff oder (C₁-C₄)-Alkyl.

Bevorzugt sind Verbindungen der allgemeinen Formel (I), worin
X bedeutet (C₁-C₄)-Alkyl,
Y bedeutet Trichlormethyl, Difluormethyl, Dichlorfluormethyl, Chlordifluormethyl, Pentafluorethyl oder Heptafluor-iso-propyl,
A und Z bedeuten jeweils (C₁-C₄)-Alkylen,
R¹ bedeutet (C₁-C₄)-Alkyl,
R² bedeutet Hydroxy,
R³ und R⁴ bedeuten unabhängig voneinander Wasserstoff oder (C₁-C₄)-Alkyl.

Besonders bevorzugt sind Verbindungen der allgemeinen Formel (I), worin
X bedeutet Methyl,
Y Chlordifluormethyl, Difluormethyl oder Pentafluorethyl,
A und Z bedeuten unabhängig voneinander jeweils CH₂ oder CH₂CH₂,
R¹ bedeutet Methyl,
R² bedeutet Hydroxy,
R³ und R⁴ bedeuten jeweils Wasserstoff,
n bedeutet 1 oder 2.

Ganz besonders bevorzugt sind Verbindungen der allgemeinen Formel (I), worin
X bedeutet Methyl,
Y Pentafluorethyl,
A und Z bedeuten unabhängig voneinander jeweils CH₂ oder CH₂CH₂,
R¹ bedeutet Methyl,
R² bedeutet Hydroxy,
R³ und R⁴ bedeuten jeweils Wasserstoff,
n bedeutet 1 oder 2.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen R² für Hydroxy steht, können beispielsweise nach dem in Schema 1 angegebenen und beispielsweise aus WO 03/084912 bekannten Verfahren durch basenkatalysierte Umsetzung eines Benzoesäurederivats (III), worin L¹ für Alkoxy oder Halogen steht, mit einem Cyclohexandion (IV) und anschließender Umlagerung hergestellt werden. Das Zwischenprodukt (V) kann auch durch direkte Umsetzung einer Benzoesäure (II) mit einem Cyclohexandion (IV) unter Zusatz eines aktivierenden Kondensations-mittels wie beispielsweise N-(3-Dimethylaminopropyl)-N'-ethyl-carbodiimidhydro-chlorid hergestellt werden.

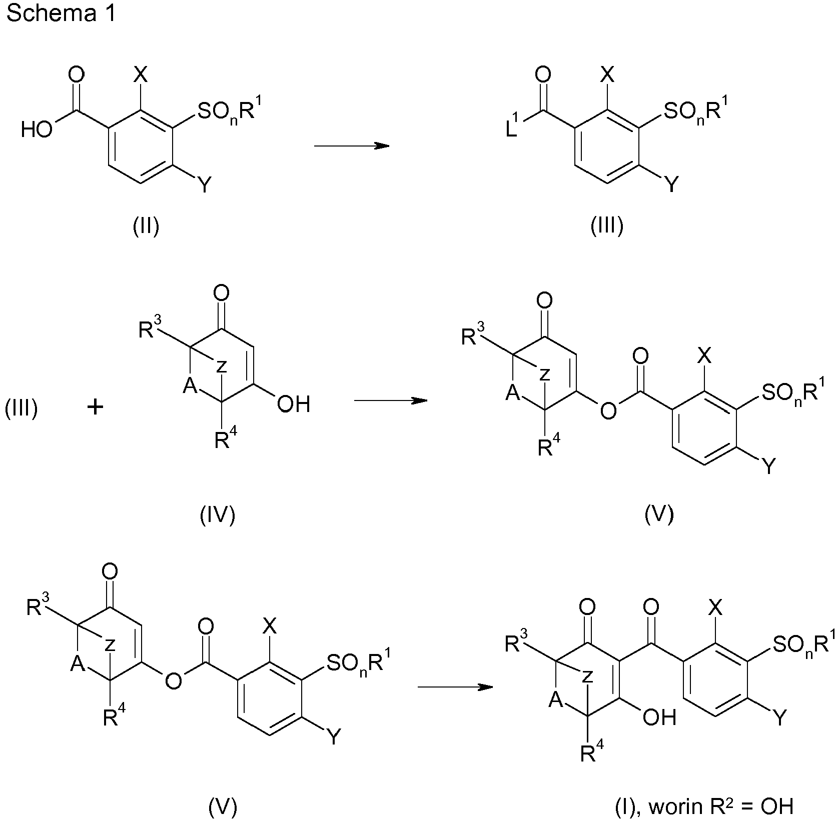

Erfindungsgemäße Verbindungen, in denen R² eine andere Bedeutung als Hydroxy hat, werden gemäß Schema 2 zweckmäßigerweise aus den nach Schema 1 erhältlichen Verbindungen hergestellt. Solche Methoden sind dem Fachmann grundsätzlich bekannt und beispielsweise in WO 03/084912 beschrieben.

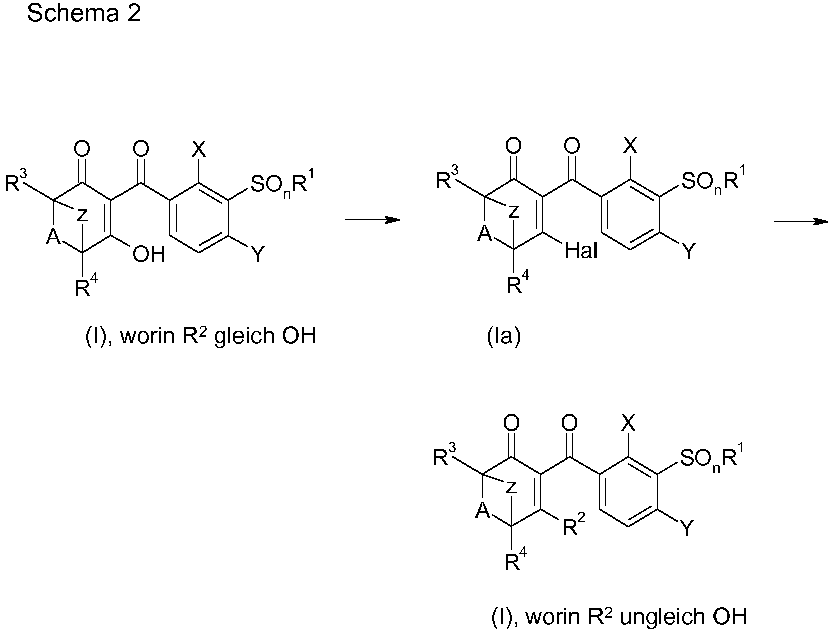

Cyclohexandione der Formel (IV) sind kommerziell erhältlich oder können gemäß dem Fachmann bekannten und beispielsweise in EP 0 338 992 und WO 2008/071405 beschriebenen Verfahren hergestellt werden. Die Herstellung der Benzoesäuren der Formel (II) ist beispiesweise aus WO 2008125214 bekannt.

Die Benzoesäuren (II) sind über eine Reihe dem Fachmann an sich bekannter synthetischer Schritte zugänglich.

Beispielsweise ist die Synthese von Benzoesäuren, die in ihrer 4-Position eine Trifluormethylgruppe als Haloalkylgruppe tragen, aus WO 2008125214 bekannt. In Abhängigkeit von der Art der Haloalkylgruppe, können die dort beschriebenen Verfahren auf die Synthese der Benzoesäuren (II) angewendet werden.

Eine weitere Möglichkeit stellt die Einführung von Haloalkylgruppen dar. Beispielsweise ist die Einführung einer Pentafluorethylgruppe in die 4-Position eines Benzonitrils in WO 2006/43064 und US 2007/185058 beschrieben. Das Benzonitril lässt sich anschließend in die entsprechende Benzoesäure überführen (US 2007/185058). Auch die Substitution am lodaromaten durch eine Haloalkylgruppe, die über die entsprechende Haloalkylkupferverbindung verläuft, ist bekannt (A. A. Kolomeitsev et. al., J. Org. Chem. 2008, 73 (7), 2607.).

Alternativ können Haloalkylgruppen aus anderen funktionellen Gruppen generiert werden. Eine Difluormethylgruppe ist beispielsweise aus der entsprechenden Formylgruppe zugänglich. Eine solche Transformation wird von C. P. Andrieux et. al. (Journal of the American Chemical Society 1997 (119), 40, 9527.) am 4-Formylbenzonitril beschrieben. Aus dem Produkt kann anschließend beispielsweise unter basischen Bedingungen die entsprechende Benzoesäure generiert werden (F. Mathey et. al., Tetrahedron 1975 (31), 391.).

Weitere synthetische Zugangsmöglichkeiten zu Benzoesäuren, die auf die Synthese der Benzoesäuren (II) angewendet werden können, sind aus WO 03/014071 bekannt.

Das Schwefelatom in der 3-Position der Benzoylgruppe muss nicht zwingend auf der Stufe der Benzoesäure oxidiert werden, um Verbindungen mit n = 1 oder 2 zu generieren; beispielsweise kann eine solche Oxidation auch auf der Stufe des Enolesters oder des Benzoylcyclohexandions zweckmäßig sein.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono-oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant).
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- gentechnisch veränderte Kulturpflanzen mit neuen Inhalts- oder Sekundärstoffen z.B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996)423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, oder gegen beliebige Kombinationen dieser Wirkstoffe, resistent sind.

Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum™ GAT™ (Glyphosate ALS Tolerant) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf ÖI- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2006 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1 H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, Glufosinateammonium, Glufosinate-P, Glufosinate-P-ammonium, Glufosinate-P-natrium, Glyphosate, Glyphosate-isopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HW-02, d. h. 1-(Dimethoxyphosphoryl)-ethyl(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyr-ammonium, Imazosulfuron, Inabenfide, Indanofan, Indaziflam, Indolessigsäure (IAA), 4-Indol-3-ylbuttersäure (IBA), lodosulfuron, Iodosulfuron-methyl-natrium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamidedihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

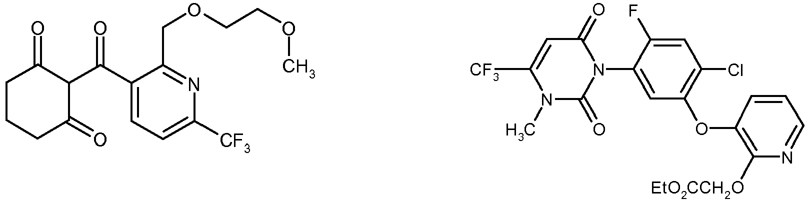

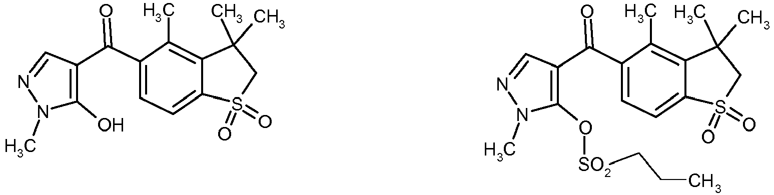

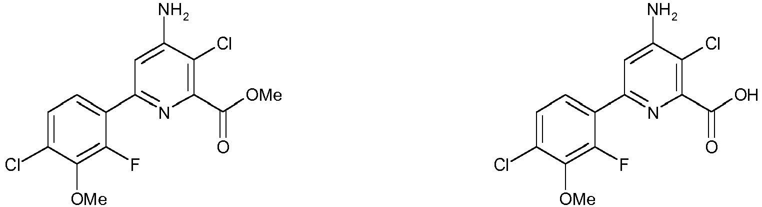

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser.

Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

Herstellung von 3-(2-Methyl-3-methylsulfinyl-4-pentafluorethyl)benzoyl)bicyclo[3.2.1] octan-2,4-dion (Tabellenbeispiel Nr. 1-153) Schritt 1: Synthese von 3-Fluor-4-(pentafluorethyl)brombenzol 13.2 g (91.9 mmol) Kupfer(I)bromid wurden in 80 ml trockenem N,N-Dimethylformamid auf-5°C abgekühlt. Unter Stickstoff wurden 14.7 g (76.7 mmol) Trimethyl(pentafluorethyl)silan zugegeben. 4.45 g (76.7 mmol) Kaliumfluorid (sprühgetrocknet) wurden innerhalb von 30 min portionsweise so zugegeben, dass die Innentemperarur unterhalb von 0 °C blieb. Das Gemisch wurde 1 h bei 0 °C gerührt und wurde dann innerhalb von 12 h auf 20 °C erwärmt. Danach wurden 10 ml trockenes 1,3-Dimethyl-2-imidazolidinon und 22 g (73.1 mmol) 3-Fluor-4-iodbrombenzol zugegeben. Der Inhalt wurde 14 h lang bei 75 °C gerührt. Anschließend wurden alle flüchtigen Bestandteile bis zur Trockene abdestilliert, wobei das Destillat in einer mit flüssigem Stickstoff gekühlten Kühlfalle aufgefangen wurde. Danach wurde das Destillat auf 20 °C aufgewärmt und in 500 ml Diethylether gelöst. Diese Lösung wurde viermal mit je 100 ml Wasser und anschließend zweimal mit je 100 ml gesättigter wässriger NaCL-Lösung gewaschen. Dann wurde die organische Phase getrocknet, und über eine Vigreux-Kolonne wurde das Lösungsmittel abgetrennt. Nach einer anschließenden Destillation des Rückstands über eine Vigreux-Kolonne wurden 16.1 g Produkt (Siedepunkt: 86 - 87 °C (75 mm Hg)) mit einer Reinheit von 99 Gew.-% gewonnen.

### Schritt 2: Synthese von 3-Fluor-4-(pentafluorethyl)benzoesäure

Eine Lösung von 16.0 g (99 Gew.-%; 54.1 mmol) 3-Fluor-4-(pentafluorethyl)-brombenzol in 110 ml Tetrahydrofuran wurde auf -10 °C abgekühlt. Hierzu wurden 36.8 ml einer 2M (73.6 mmol) Lösung von Isopropylmagnesiumchlorid innerhalb von 30 min tropfenweise zugegeben. Das Gemisch wurde 2.5 h lang bei -10 °C gerührt und anschließend vorsichtig auf einen Überschuss von Trockeneis gegeben. Dann wurden 130 ml Wasser zugegeben, und das Gemisch wurde danach einmal mit 10 ml Diethylether extrahiert. Die wässrige Phase wurde mit halbkonzentrierter Salzsäure angesäuert und dann viermal mit je 150 ml Diethylether extrahiert. Die vereinigten organischen Phasen wurden getrocknet, und das Filtrat wurde vom Lösungsmittel befreit. Der Rückstand wurde aus Chloroform umkristallisiert, wobei 10.7 g Produkt mit einer Reinheit von 98 Gew.-% gewonnen wurden.

### Schritt 3: Synthese von 3-Fluor-2-methyl-4-(pentafluorethyl)benzoesäure

5.00 g (98 Gew.-%; 19.0 mmol) 3-Fluor-4-(pentafluorethyl)benzoesäure wurden in 40 ml trockenem Tetrahydrofuran vorgelegt. 4.73 g (40.7 mmol) 1,2-Bis(dimethylamino)ethan wurden zugegeben, und das Gemisch wurde dann auf-40 °C abgekühlt. Anschließend wurden 16.3 ml einer 2.5 M Lösung (40.7 mmol) von n-Butyllithium in Hexan so zugetropft, dass sich die Temperatur in einem Bereich von -33 °C bis -35 °C befand. Der Inhalt wurde 4 h bei einer Temperatur von -35 °C gerührt. Anschließend wurde eine Lösung von 8.25 g (58.1 mmol) lodmethan in 10 ml trockenem Tetrahydrofuran zugetropft, und das Gemisch wurde 1 h bei einer Temperatur von -35 °C gerührt. Danach wurde der Inhalt auf Raumtemperatur (RT) erwärmt und 16 h bei dieser Temperatur gerührt. Zur Aufarbeitung wurden 50 ml 2M Salzsäure vorsichtig zugegeben, und anschließend wurde die wässrige Phase mit Diethylether extrahiert. Die vereinigten organischen Phasen wurden getrocknet und am Rotationsverdampfer von den Lösungsmitteln befreit. Der Rückstand wurde in n-Heptan verrührt und danach filtriert. Es wurden 2.33 g Rückstand als sauberes Produkt gewonnen. Das Filtrat wurde vom Lösungsmittel befreit, und der Rückstand wurde erneut in n-Heptan verrührt. Die Filtration ergab 590 mg Rückstand, der als Produkt mit einer Reinheit von 70 Gew.-% identifiziert wurde.

### Schritt 4: Synthese von 3-Fluor-2-methyl-4-(pentafluorethyl)benzoesäuremethylester

2.33 g (8.56 mmol) 3-Fluor-2-methyl-4-(pentafluorethyl)benzoesäure wurden in 30 ml Methanol vorgelegt und mit 2 ml konz. Schwefelsäure versetzt. Das Gemisch wurde anschließend so lange unter Rückfluss erhitzt, bis die HPLC-Kontrolle einen vollständigen Umsatz anzeigte. Der Inhalt wurde am Rotationsverdampfer vom Lösungsmittel befreit, und der Rückstand wurde in Wasser aufgenommen. Das Gemisch wurde zweimal mit Essigsäureethylester extrahiert, und die vereinigten organischen Phasen wurden einmal mit gesättigter wässriger Natriumhydrogencarbonat-Lösung gewaschen. Die organische Phase wurde getrocknet und vom Lösungsmittel befreit. Als Rückstand wurden 2.1 g sauberes Produkt gewonnen.

### Schritt 5: Synthese von 2-Methyl-3-(methylthio)-4-(pentafluorethyl)benzoesäuremethylester

2.1 g (7.34 mmol) 3-Fluor-2-methyl-4-(pentafluorethyl)benzoesäuremethylester wurden in 20 ml DMF vorgelegt und dann mit 758 mg (95 Gew.-%; 10.3 mmol) Natriumthiomethylat versetzt. Das Gemisch wurde 2 h bei RT gerührt und anschließend vom Lösungsmittel befreit. Der Rückstand wurde in Essigsäureethylester und Wasser aufgenommen, die wässrige Phase wurde zweimal mit Essigsäureethylester extrahiert und schließlich wurden die vereinigten organischen Phasen getrocknet und vom Lösungsmittel befreit. Der Rückstand wurde chromatographisch gereinigt, wobei 1.28 g sauberes Produkt gewonnen wurden.

### Schritt 6: Synthese von 2-Methyl-3-(methylthio)-4-(pentafluorethyl)benzoesäure (Nr. 6-145)

530 mg (1.69 mmol) 2-Methyl-3-(methylthio)-4-(pentafluorethyl)benzoesäuremethylester wurden in 10 ml Methanol vorgelegt und mit 0.5 ml 20 %-iger Natronlauge versetzt. Der Inhalt wurde so lange bei RT gerührt, bis eine dünnschichtchromatographische Reaktionskontrolle eine vollständige Umsetzung anzeigte. Das Gemisch wurde vom Lösungsmittel befreit, und der Rückstand wurde in wenig Wasser aufgenommen. Das Gemisch wurde mit 1 M Salzsäure angesäuert, anschließend 5 min gerührt nd schließlich filtriert. Als Rückstand wurden 470 mg sauberes Produkt gewonnen.

### Schritt 7: Synthese von 4-Oxobicyclo[3.2.1]oct-2-en-2-yl(2-methyl-3-methylthio-4-(pentafluorethyl)benzoat)

235 mg (0.783 mmol) 2-Methyl-3-(methylthio)-4-(pentafluorethyl)benzoesäure wurden in 20 ml trockenem Dichlormethan vorgelegt und nacheinander mit 129 mg (1.02 mmol) Oxalylchlorid sowie zwei Tropfen N,N-Dimethylformamid versetzt. Nach Ende der Gasabspaltung wurde das Gemisch noch für 10 min unter Rückfluss erhitzt. Danach wurde der Inhalt abgekühlt und am Rotationsverdampfer eingeengt. Der Rückstand wurde in 20 ml trockenem Dichlormethan aufgenommen und mit 119 mg (0.861 mmol) Bicyclo[3.2.1]octan-2,4-dion sowie einer katalytischen Menge DMAP versetzt. Anschließend wurden 158 mg (1.57 mmol) Triethylamin zugetropft. Das Gemisch wurde 16 h bei RT gerührt. Zur Aufarbeitung wurden 3 ml 1N Salzsäure zugegeben. Nach der Phasentrennung wurde die organische Phase vom Lösungsmittel befreit, und der Rückstand wurde schließlich chromatographisch gereinigt. Es wurden 280 mg Produkt mit einer Reinheit von 90 Gew.-% gewonnen.

### Schritt 8: Synthese von 4-Oxobicyclo[3.2.1]oct-2-en-2-yl(2-methyl-3-methylsulfinyl-4-pentafluorethylbenzoat)

280 mg (90 Gew.-%; 0.599 mmol) 4-Oxobicyclo[3.2.1]oct-2-en-2-yl(2-methyl-3-methylthio-4-pentafluorethylbenzoat) wurden in 20 ml Dichlormethan vorgelegt und mit 134 mg (77 Gew.-%; 0.599 mmol) Metachlorperbenzoesäure versetzt. Das Gemisch wurde bei RT so lange gerührt, bis eine dünnschichtchromatographische Reaktionskontrolle eine vollständige Umsetzung anzeigte. Zur Aufarbeitung wurde der Inhalt einmal mit 10 %-iger wässriger Natriumhydrogensulfit-Lösung, anschließend zweimal mit gesättigter wässriger Natriumhydrogencarbonat-Lösung gewaschen. Schließlich wurde die organische Phase vom Lösungsmittel befreit. Als Rückstand wurden 250 mg Produkt mit einer Reinheit von 95 Gew.-% gewonnen.

### Schritt 9: Synthese von 3-(2'-Methyl-3-methylsulfinyl-4-pentafluorethylbenzoyl)bicyclo[3.2.1]octan-2,4-dion (Nr. 1-153)

250 mg (95 Gew.-%; 0.544 mmol) 4-Oxobicyclo[3.2.1]oct-2-en-2-yl(2-methyl-3-methylsulfinyl-4-pentafluorethylbenzoat) wurden in 15 ml Acetonitril vorgelegt und nacheinander mit 110 mg (1.09 mmol) Triethylamin, einer katalytischen Menge Kaliumcyanid sowie acht Tropfen Trimetyhlsilylcyanid versetzt. Das Gemisch wurde 16 h bei RT gerührt. Zur Aufarbeitung wurde der Inhalt eingeengt, und der Rückstand wurde in 15 ml Dichlormethan aufgenommen. Das Gemisch wurde mit 3 ml 1 M HCl versetzt, und nach der Phasentrennung wurde die organische Phase vom Lösungsmittel befreit. Der Rückstand wurde schließlich chromatographisch gereinigt, wobei 86.4 mg Produkt mit einer Reinheit von 95 Gew.-% gewonnen wurden.

Die in nachfolgenden Tabellen aufgeführten Beispiele wurden analog oben genannten Methoden hergestellt beziehungsweise sind analog oben genannten Methoden erhältlich. Diese Verbindungen sind ganz besonders bevorzugt.

Die verwendeten Abkürzungen bedeuten:
Bu = Butyl Et = Ethyl Me = Methyl Pr = Propyl i = iso s = sekundär t = tertiär Ph = Phenyl

**Tabelle 1: Erfindungsgemäße Verbindungen der allgemeinen Formel (I), worin R² für Hydroxy, A für (CH₂) und Z für (CH₂)₂ stehen, sowie R³ und R⁴ jeweils Wasserstoff bedeuten**

| | | | | | |
|---|---|---|---|---|---|
| 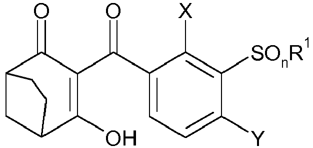 | | | | | |

| Nr. | X | R¹ | n | Y | Physikalische Daten: ¹H-NMR: δ [CDCl₃] |
|---|---|---|---|---|---|
| 1-1 | Me | Me | 0 | CF₂Cl | |
| 1-2 | Me | Et | 0 | CF₂Cl | |
| 1-3 | Me | n-Pr | 0 | CF₂Cl | |
| 1-4 | Me | i-Pr | 0 | CF₂Cl | |
| 1-5 | Me | n-Bu | 0 | CF₂Cl | |
| 1-6 | Me | i-Bu | 0 | CF₂Cl | |
| 1-7 | Me | s-Bu | 0 | CF₂Cl | |
| 1-8 | Me | t-Bu | 0 | CF₂Cl | |
| 1-9 | Me | Me | 1 | CF₂Cl | |
| 1-10 | Me | Et | 1 | CF₂Cl | |
| 1-11 | Me | n-Pr | 1 | CF₂Cl | |
| 1-12 | Me | i-Pr | 1 | CF₂Cl | |
| 1-13 | Me | n-Bu | 1 | CF₂Cl | |
| 1-14 | Me | i-Bu | 1 | CF₂Cl | |
| 1-15 | Me | s-Bu | 1 | CF₂Cl | |
| 1-16 | Me | t-Bu | 1 | CF₂Cl | |
| 1-17 | Me | Me | 2 | CF₂Cl | |
| 1-18 | Me | Et | 2 | CF₂Cl | |
| 1-19 | Me | n-Pr | 2 | CF₂Cl | |
| 1-20 | Me | i-Pr | 2 | CF₂Cl | |
| 1-21 | Me | n-Bu | 2 | CF₂Cl | |
| 1-22 | Me | i-Bu | 2 | CF₂Cl | |
| 1-23 | Me | s-Bu | 2 | CF₂Cl | |
| 1-24 | Me | t-Bu | 2 | CF₂Cl | |
| 1-25 | Et | Me | 0 | CF₂Cl | |
| 1-26 | Et | Et | 0 | CF₂Cl | |
| 1-27 | Et | n-Pr | 0 | CF₂Cl | |
| 1-28 | Et | i-Pr | 0 | CF₂Cl | |
| 1-29 | Et | n-Bu | 0 | CF₂Cl | |
| 1-30 | Et | i-Bu | 0 | CF₂Cl | |
| 1-31 | Et | s-Bu | 0 | CF₂Cl | |
| 1-32 | Et | t-Bu | 0 | CF₂Cl | |
| 1-33 | Et | Me | 1 | CF₂Cl | |
| 1-34 | Et | Et | 1 | CF₂Cl | |
| 1-35 | Et | n-Pr | 1 | CF₂Cl | |
| 1-36 | Et | i-Pr | 1 | CF₂Cl | |
| 1-37 | Et | n-Bu | 1 | CF₂Cl | |
| 1-38 | Et | i-Bu | 1 | CF₂Cl | |
| 1-39 | Et | s-Bu | 1 | CF₂Cl | |
| 1-40 | Et | t-Bu | 1 | CF₂Cl | |
| 1-41 | Et | Me | 2 | CF₂Cl | |
| 1-42 | Et | Et | 2 | CF₂Cl | |
| 1-43 | Et | n-Pr | 2 | CF₂Cl | |
| 1-44 | Et | i-Pr | 2 | CF₂Cl | |
| 1-45 | Et | n-Bu | 2 | CF₂Cl | |
| 1-46 | Et | i-Bu | 2 | CF₂Cl | |
| 1-47 | Et | s-Bu | 2 | CF₂Cl | |
| 1-48 | Et | t-Bu | 2 | CF₂Cl | |
| 1-49 | Me | Me | 0 | CFCl₂ | |
| 1-50 | Me | Et | 0 | CFCl₂ | |
| 1-51 | Me | n-Pr | 0 | CFCl₂ | |
| 1-52 | Me | i-Pr | 0 | CFCl₂ | |
| 1-53 | Me | n-Bu | 0 | CFCl₂ | |
| 1-54 | Me | i-Bu | 0 | CFCl₂ | |
| 1-55 | Me | s-Bu | 0 | CFCl₂ | |
| 1-56 | Me | t-Bu | 0 | CFCl₂ | |
| 1-57 | Me | Me | 1 | CFCl₂ | |
| 1-58 | Me | Et | 1 | CFCl₂ | |
| 1-59 | Me | n-Pr | 1 | CFCl₂ | |
| 1-60 | Me | i-Pr | 1 | CFCl₂ | |
| 1-61 | Me | n-Bu | 1 | CFCl₂ | |
| 1-62 | Me | i-Bu | 1 | CFCl₂ | |
| 1-63 | Me | s-Bu | 1 | CFCl₂ | |
| 1-64 | Me | t-Bu | 1 | CFCl₂ | |
| 1-65 | Me | Me | 2 | CFCl₂ | |
| 1-66 | Me | Et | 2 | CFCl₂ | |
| 1-67 | Me | n-Pr | 2 | CFCl₂ | |
| 1-68 | Me | i-Pr | 2 | CFCl₂ | |
| 1-69 | Me | n-Bu | 2 | CFCl₂ | |
| 1-70 | Me | i-Bu | 2 | CFCl₂ | |
| 1-71 | Me | s-Bu | 2 | CFCl₂ | |
| 1-72 | Me | t-Bu | 2 | CFCl₂ | |
| 1-73 | Et | Me | 0 | CFCl₂ | |
| 1-74 | Et | Et | 0 | CFCl₂ | |
| 1-75 | Et | n-Pr | 0 | CFCl₂ | |
| 1-76 | Et | i-Pr | 0 | CFCl₂ | |
| 1-77 | Et | n-Bu | 0 | CFCl₂ | |
| 1-78 | Et | i-Bu | 0 | CFCl₂ | |
| 1-79 | Et | s-Bu | 0 | CFCl₂ | |
| 1-80 | Et | t-Bu | 0 | CFCl₂ | |
| 1-81 | Et | Me | 1 | CFCl₂ | |
| 1-82 | Et | Et | 1 | CFCl₂ | |
| 1-83 | Et | n-Pr | 1 | CFCl₂ | |
| 1-84 | Et | i-Pr | 1 | CFCl₂ | |
| 1-85 | Et | n-Bu | 1 | CFCl₂ | |
| 1-86 | Et | i-Bu | 1 | CFCl₂ | |
| 1-87 | Et | s-Bu | 1 | CFCl₂ | |
| 1-88 | Et | t-Bu | 1 | CFCl₂ | |
| 1-89 | Et | Me | 2 | CFCl₂ | |
| 1-90 | Et | Et | 2 | CFCl₂ | |
| 1-91 | Et | n-Pr | 2 | CFCl₂ | |
| 1-92 | Et | i-Pr | 2 | CFCl₂ | |
| 1-93 | Et | n-Bu | 2 | CFCl₂ | |
| 1-94 | Et | i-Bu | 2 | CFCl₂ | |
| 1-95 | Et | s-Bu | 2 | CFCl₂ | |
| 1-96 | Et | t-Bu | 2 | CFCl₂ | |
| 1-97 | Me | Me | 0 | CCl₃ | |
| 1-98 | Me | Et | 0 | CCl₃ | |
| 1-99 | Me | n-Pr | 0 | CCl₃ | |
| 1-100 | Me | i-Pr | 0 | CCl₃ | |
| 1-101 | Me | n-Bu | 0 | CCl₃ | |
| 1-102 | Me | i-Bu | 0 | CCl₃ | |
| 1-103 | Me | s-Bu | 0 | CCl₃ | |
| 1-104 | Me | t-Bu | 0 | CCl₃ | |
| 1-105 | Me | Me | 1 | CCl₃ | |
| 1-106 | Me | Et | 1 | CCl₃ | |
| 1-107 | Me | n-Pr | 1 | CCl₃ | |
| 1-108 | Me | i-Pr | 1 | CCl₃ | |
| 1-109 | Me | n-Bu | 1 | CCl₃ | |
| 1-110 | Me | i-Bu | 1 | CCl₃ | |
| 1-111 | Me | s-Bu | 1 | CCl₃ | |
| 1-112 | Me | t-Bu | 1 | CCl₃ | |
| 1-113 | Me | Me | 2 | CCl₃ | |
| 1-114 | Me | Et | 2 | CCl₃ | |
| 1-115 | Me | n-Pr | 2 | CCl₃ | |
| 1-116 | Me | i-Pr | 2 | CCl₃ | |
| 1-117 | Me | n-Bu | 2 | CCl₃ | |
| 1-118 | Me | i-Bu | 2 | CCl₃ | |
| 1-119 | Me | s-Bu | 2 | CCl₃ | |
| 1-120 | Me | t-Bu | 2 | CCl₃ | |
| 1-121 | Et | Me | 0 | CCl₃ | |
| 1-122 | Et | Et | 0 | CCl₃ | |
| 1-123 | Et | n-Pr | 0 | CCl₃ | |
| 1-124 | Et | i-Pr | 0 | CCl₃ | |
| 1-125 | Et | n-Bu | 0 | CCl₃ | |
| 1-126 | Et | i-Bu | 0 | CCl₃ | |
| 1-127 | Et | s-Bu | 0 | CCl₃ | |
| 1-128 | Et | t-Bu | 0 | CCl₃ | |
| 1-129 | Et | Me | 1 | CCl₃ | |
| 1-130 | Et | Et | 1 | CCl₃ | |
| 1-131 | Et | n-Pr | 1 | CCl₃ | |
| 1-132 | Et | i-Pr | 1 | CCl₃ | |
| 1-133 | Et | n-Bu | 1 | CCl₃ | |
| 1-134 | Et | i-Bu | 1 | CCl₃ | |
| 1-135 | Et | s-Bu | 1 | CCl₃ | |
| 1-136 | Et | t-Bu | 1 | CCl₃ | |
| 1-137 | Et | Me | 2 | CCl₃ | |
| 1-138 | Et | Et | 2 | CCl₃ | |
| 1-139 | Et | n-Pr | 2 | CCl₃ | |
| 1-140 | Et | i-Pr | 2 | CCl₃ | |
| 1-141 | Et | n-Bu | 2 | CCl₃ | |
| 1-142 | Et | i-Bu | 2 | CCl₃ | |
| 1-143 | Et | s-Bu | 2 | CCl₃ | |
| 1-144 | Et | t-Bu | 2 | CCl₃ | |
| 1-145 | Me | Me | 0 | C₂F₅ | |
| 1-146 | Me | Et | 0 | C₂F₅ | |
| 1-147 | Me | n-Pr | 0 | C₂F₅ | |
| 1-148 | Me | i-Pr | 0 | C₂F₅ | |
| 1-149 | Me | n-Bu | 0 | C₂F₅ | |
| 1-150 | Me | i-Bu | 0 | C₂F₅ | |
| 1-151 | Me | s-Bu | 0 | C₂F₅ | |
| 1-152 | Me | t-Bu | 0 | C₂F₅ | |
| 1-153 | Me | Me | 1 | C₂F₅ | 17.21 (s + s, 1H), 7.53 (d, 1H), 7.23 (d, 1H), 3.17 (m, 1H), 3.02 (s, 3H), 2.88 (m, 1H), 2.74 (s+s, 3H), 2.30 - 2.09 (m, 3H), 2.09 - 2.00 (m, 1H), 1.80 - 1.62 (m, 2H) |
| 1-154 | Me | Et | 1 | C₂F₅ | |
| 1-155 | Me | n-Pr | 1 | C₂F₅ | |
| 1-156 | Me | i-Pr | 1 | C₂F₅ | |
| 1-157 | Me | n-Bu | 1 | C₂F₅ | |
| 1-158 | Me | i-Bu | 1 | C₂F₅ | |
| 1-159 | Me | s-Bu | 1 | C₂F₅ | |
| 1-160 | Me | t-Bu | 1 | C₂F₅ | |
| 1-161 | Me | Me | 2 | C₂F₅ | 17.08 (s, 1H), 7.67 (d, 1H), 7.32 (d, 1H), 3.26 (s, 3H), 3.18 (m, 1H), 2.88 (m, 1H), 2.60 (s, 3H), 2.31 - 2.10 (m, 3H), 2.03 (m, 1H), 1.81 - 1.67 (m, 2H) |
| 1-162 | Me | Et | 2 | C₂F₅ | |
| 1-163 | Me | n-Pr | 2 | C₂F₅ | |
| 1-164 | Me | i-Pr | 2 | C₂F₅ | |
| 1-165 | Me | n-Bu | 2 | C₂F₅ | |
| 1-166 | Me | i-Bu | 2 | C₂F₅ | |
| 1-167 | Me | s-Bu | 2 | C₂F₅ | |
| 1-168 | Me | t-Bu | 2 | C₂F₅ | |
| 1-169 | Et | Me | 0 | C₂F₅ | |
| 1-170 | Et | Et | 0 | C₂F₅ | |
| 1-171 | Et | n-Pr | 0 | C₂F₅ | |
| 1-172 | Et | i-Pr | 0 | C₂F₅ | |
| 1-173 | Et | n-Bu | 0 | C₂F₅ | |
| 1-174 | Et | i-Bu | 0 | C₂F₅ | |
| 1-175 | Et | s-Bu | 0 | C₂F₅ | |
| 1-176 | Et | t-Bu | 0 | C₂F₅ | |
| 1-177 | Et | Me | 1 | C₂F₅ | |
| 1-178 | Et | Et | 1 | C₂F₅ | |
| 1-179 | Et | n-Pr | 1 | C₂F₅ | |
| 1-180 | Et | i-Pr | 1 | C₂F₅ | |
| 1-181 | Et | n-Bu | 1 | C₂F₅ | |
| 1-182 | Et | i-Bu | 1 | C₂F₅ | |
| 1-183 | Et | s-Bu | 1 | C₂F₅ | |
| 1-184 | Et | t-Bu | 1 | C₂F₅ | |
| 1-185 | Et | Me | 2 | C₂F₅ | |
| 1-186 | Et | Et | 2 | C₂F₅ | |
| 1-187 | Et | n-Pr | 2 | C₂F₅ | |
| 1-188 | Et | i-Pr | 2 | C₂F₅ | |
| 1-189 | Et | n-Bu | 2 | C₂F₅ | |
| 1-190 | Et | i-Bu | 2 | C₂F₅ | |
| 1-191 | Et | s-Bu | 2 | C₂F₅ | |
| 1-192 | Et | t-Bu | 2 | C₂F₅ | |
| 1-193 | Me | Me | 0 | CF(CF₃)₂ | |
| 1-194 | Me | Et | 0 | CF(CF₃)₂ | |
| 1-195 | Me | n-Pr | 0 | CF(CF₃)₂ | |
| 1-196 | Me | i-Pr | 0 | CF(CF₃)₂ | |
| 1-197 | Me | n-Bu | 0 | CF(CF₃)₂ | |
| 1-198 | Me | i-Bu | 0 | CF(CF₃)₂ | |
| 1-199 | Me | s-Bu | 0 | CF(CF₃)₂ | |
| 1-200 | Me | t-Bu | 0 | CF(CF₃)₂ | |
| 1-201 | Me | Me | 1 | CF(CF₃)₂ | |
| 1-202 | Me | Et | 1 | CF(CF₃)₂ | |
| 1-203 | Me | n-Pr | 1 | CF(CF₃)₂ | |
| 1-204 | Me | i-Pr | 1 | CF(CF₃)₂ | |
| 1-205 | Me | n-Bu | 1 | CF(CF₃)₂ | |
| 1-206 | Me | i-Bu | 1 | CF(CF₃)₂ | |
| 1-207 | Me | s-Bu | 1 | CF(CF₃)₂ | |
| 1-208 | Me | t-Bu | 1 | CF(CF₃)₂ | |
| 1-209 | Me | Me | 2 | CF(CF₃)₂ | |
| 1-210 | Me | Et | 2 | CF(CF₃)₂ | |
| 1-211 | Me | n-Pr | 2 | CF(CF₃)₂ | |
| 1-212 | Me | i-Pr | 2 | CF(CF₃)₂ | |
| 1-213 | Me | n-Bu | 2 | CF(CF₃)₂ | |
| 1-214 | Me | i-Bu | 2 | CF(CF₃)₂ | |
| 1-215 | Me | s-Bu | 2 | CF(CF₃)₂ | |
| 1-216 | Me | t-Bu | 2 | CF(CF₃)₂ | |
| 1-217 | Et | Me | 0 | CF(CF₃)₂ | |
| 1-218 | Et | Et | 0 | CF(CF₃)₂ | |
| 1-219 | Et | n-Pr | 0 | CF(CF₃)₂ | |
| 1-220 | Et | i-Pr | 0 | CF(CF₃)₂ | |
| 1-221 | Et | n-Bu | 0 | CF(CF₃)₂ | |
| 1-222 | Et | i-Bu | 0 | CF(CF₃)₂ | |
| 1-223 | Et | s-Bu | 0 | CF(CF₃)₂ | |
| 1-224 | Et | t-Bu | 0 | CF(CF₃)₂ | |
| 1-225 | Et | Me | 1 | CF(CF₃)₂ | |
| 1-226 | Et | Et | 1 | CF(CF₃)₂ | |
| 1-227 | Et | n-Pr | 1 | CF(CF₃)₂ | |
| 1-228 | Et | i-Pr | 1 | CF(CF₃)₂ | |
| 1-229 | Et | n-Bu | 1 | CF(CF₃)₂ | |
| 1-230 | Et | i-Bu | 1 | CF(CF₃)₂ | |
| 1-231 | Et | s-Bu | 1 | CF(CF₃)₂ | |
| 1-232 | Et | t-Bu | 1 | CF(CF₃)₂ | |
| 1-233 | Et | Me | 2 | CF(CF₃)₂ | |
| 1-234 | Et | Et | 2 | CF(CF₃)₂ | |
| 1-235 | Et | n-Pr | 2 | CF(CF₃)₂ | |
| 1-236 | Et | i-Pr | 2 | CF(CF₃)₂ | |
| 1-237 | Et | n-Bu | 2 | CF(CF₃)₂ | |
| 1-238 | Et | i-Bu | 2 | CF(CF₃)₂ | |
| 1-239 | Et | s-Bu | 2 | CF(CF₃)₂ | |
| 1-240 | Et | t-Bu | 2 | CF(CF₃)₂ | |
| 1-241 | Me | Me | 0 | CHF₂ | |
| 1-242 | Me | Et | 0 | CHF₂ | |
| 1-243 | Me | n-Pr | 0 | CHF₂ | |
| 1-244 | Me | i-Pr | 0 | CHF₂ | |
| 1-245 | Me | n-Bu | 0 | CHF₂ | |
| 1-246 | Me | i-Bu | 0 | CHF₂ | |
| 1-247 | Me | s-Bu | 0 | CHF₂ | |
| 1-248 | Me | t-Bu | 0 | CHF₂ | |
| 1-249 | Me | Me | 1 | CHF₂ | |
| 1-250 | Me | Et | 1 | CHF₂ | |
| 1-251 | Me | n-Pr | 1 | CHF₂ | |
| 1-252 | Me | i-Pr | 1 | CHF₂ | |
| 1-253 | Me | n-Bu | 1 | CHF₂ | |
| 1-254 | Me | i-Bu | 1 | CHF₂ | |
| 1-255 | Me | s-Bu | 1 | CHF₂ | |
| 1-256 | Me | t-Bu | 1 | CHF₂ | |
| 1-257 | Me | Me | 2 | CHF₂ | |
| 1-258 | Me | Et | 2 | CHF₂ | |
| 1-259 | Me | n-Pr | 2 | CHF₂ | |
| 1-260 | Me | i-Pr | 2 | CHF₂ | |
| 1-261 | Me | n-Bu | 2 | CHF₂ | |
| 1-262 | Me | i-Bu | 2 | CHF₂ | |
| 1-263 | Me | s-Bu | 2 | CHF₂ | |
| 1-264 | Me | t-Bu | 2 | CHF₂ | |
| 1-265 | Et | Me | 0 | CHF₂ | |
| 1-266 | Et | Et | 0 | CHF₂ | |
| 1-267 | Et | n-Pr | 0 | CHF₂ | |
| 1-268 | Et | i-Pr | 0 | CHF₂ | |
| 1-269 | Et | n-Bu | 0 | CHF₂ | |
| 1-270 | Et | i-Bu | 0 | CHF₂ | |
| 1-271 | Et | s-Bu | 0 | CHF₂ | |
| 1-272 | Et | t-Bu | 0 | CHF₂ | |
| 1-273 | Et | Me | 1 | CHF₂ | |
| 1-274 | Et | Et | 1 | CHF₂ | |
| 1-275 | Et | n-Pr | 1 | CHF₂ | |
| 1-276 | Et | i-Pr | 1 | CHF₂ | |
| 1-277 | Et | n-Bu | 1 | CHF₂ | |
| 1-278 | Et | i-Bu | 1 | CHF₂ | |
| 1-279 | Et | s-Bu | 1 | CHF₂ | |
| 1-280 | Et | t-Bu | 1 | CHF₂ | |
| 1-281 | Et | Me | 2 | CHF₂ | |
| 1-282 | Et | Et | 2 | CHF₂ | |
| 1-283 | Et | n-Pr | 2 | CHF₂ | |
| 1-284 | Et | i-Pr | 2 | CHF₂ | |
| 1-285 | Et | n-Bu | 2 | CHF₂ | |
| 1-286 | Et | i-Bu | 2 | CHF₂ | |
| 1-287 | Et | s-Bu | 2 | CHF₂ | |
| 1-288 | Et | t-Bu | 2 | CHF₂ | |

**Tabelle 2: Erfindungsgemäße Verbindungen der allgemeinen Formel (I), worin R² für Hydroxy, A für Sauerstoff, Z für (CH₂)₂, R³ für Methyl, sowie R⁴ für Wasserstoff stehen**

| | | | | | |
|---|---|---|---|---|---|
| 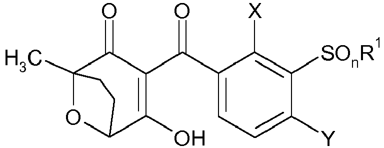 | | | | | |

| Nr. | X | R¹ | n | Y | Physikalische Daten: ¹H-NMR: δ [CDCl₃] |
|---|---|---|---|---|---|
| 2-1 | Me | Me | 0 | CF₂Cl | |
| 2-2 | Me | Et | 0 | CF₂Cl | |
| 2-3 | Me | n-Pr | 0 | CF₂Cl | |
| 2-4 | Me | i-Pr | 0 | CF₂Cl | |
| 2-5 | Me | n-Bu | 0 | CF₂Cl | |
| 2-6 | Me | i-Bu | 0 | CF₂Cl | |
| 2-7 | Me | s-Bu | 0 | CF₂Cl | |
| 2-8 | Me | t-Bu | 0 | CF₂Cl | |
| 2-9 | Me | Me | 1 | CF₂Cl | |
| 2-10 | Me | Et | 1 | CF₂Cl | |
| 2-11 | Me | n-Pr | 1 | CF₂Cl | |
| 2-12 | Me | i-Pr | 1 | CF₂Cl | |
| 2-13 | Me | n-Bu | 1 | CF₂Cl | |
| 2-14 | Me | i-Bu | 1 | CF₂Cl | |
| 2-15 | Me | s-Bu | 1 | CF₂Cl | |
| 2-16 | Me | t-Bu | 1 | CF₂Cl | |
| 2-17 | Me | Me | 2 | CF₂Cl | |
| 2-18 | Me | Et | 2 | CF₂Cl | |
| 2-19 | Me | n-Pr | 2 | CF₂Cl | |
| 2-20 | Me | i-Pr | 2 | CF₂Cl | |
| 2-21 | Me | n-Bu | 2 | CF₂Cl | |
| 2-22 | Me | i-Bu | 2 | CF₂Cl | |
| 2-23 | Me | s-Bu | 2 | CF₂Cl | |
| 2-24 | Me | t-Bu | 2 | CF₂Cl | |
| 2-25 | Et | Me | 0 | CF₂Cl | |
| 2-26 | Et | Et | 0 | CF₂Cl | |
| 2-27 | Et | n-Pr | 0 | CF₂Cl | |
| 2-28 | Et | i-Pr | 0 | CF₂Cl | |
| 2-29 | Et | n-Bu | 0 | CF₂Cl | |
| 2-30 | Et | i-Bu | 0 | CF₂Cl | |
| 2-31 | Et | s-Bu | 0 | CF₂Cl | |
| 2-32 | Et | t-Bu | 0 | CF₂Cl | |
| 2-33 | Et | Me | 1 | CF₂Cl | |
| 2-34 | Et | Et | 1 | CF₂Cl | |
| 2-35 | Et | n-Pr | 1 | CF₂Cl | |
| 2-36 | Et | i-Pr | 1 | CF₂Cl | |
| 2-37 | Et | n-Bu | 1 | CF₂Cl | |
| 2-38 | Et | i-Bu | 1 | CF₂Cl | |
| 2-39 | Et | s-Bu | 1 | CF₂Cl | |
| 2-40 | Et | t-Bu | 1 | CF₂Cl | |
| 2-41 | Et | Me | 2 | CF₂Cl | |
| 2-42 | Et | Et | 2 | CF₂Cl | |
| 2-43 | Et | n-Pr | 2 | CF₂Cl | |
| 2-44 | Et | i-Pr | 2 | CF₂Cl | |
| 2-45 | Et | n-Bu | 2 | CF₂Cl | |
| 2-46 | Et | i-Bu | 2 | CF₂Cl | |
| 2-47 | Et | s-Bu | 2 | CF₂Cl | |
| 2-48 | Et | t-Bu | 2 | CF₂Cl | |
| 2-49 | Me | Me | 0 | CFCl₂ | |
| 2-50 | Me | Et | 0 | CFCl₂ | |
| 2-51 | Me | n-Pr | 0 | CFCl₂ | |
| 2-52 | Me | i-Pr | 0 | CFCl₂ | |
| 2-53 | Me | n-Bu | 0 | CFCl₂ | |
| 2-54 | Me | i-Bu | 0 | CFCl₂ | |
| 2-55 | Me | s-Bu | 0 | CFCl₂ | |
| 2-56 | Me | t-Bu | 0 | CFCl₂ | |
| 2-57 | Me | Me | 1 | CFCl₂ | |
| 2-58 | Me | Et | 1 | CFCl₂ | |
| 2-59 | Me | n-Pr | 1 | CFCl₂ | |
| 2-60 | Me | i-Pr | 1 | CFCl₂ | |
| 2-61 | Me | n-Bu | 1 | CFCl₂ | |
| 2-62 | Me | i-Bu | 1 | CFCl₂ | |
| 2-63 | Me | s-Bu | 1 | CFCl₂ | |
| 2-64 | Me | t-Bu | 1 | CFCl₂ | |
| 2-65 | Me | Me | 2 | CFCl₂ | |
| 2-66 | Me | Et | 2 | CFCl₂ | |
| 2-67 | Me | n-Pr | 2 | CFCl₂ | |
| 2-68 | Me | i-Pr | 2 | CFCl₂ | |
| 2-69 | Me | n-Bu | 2 | CFCl₂ | |
| 2-70 | Me | i-Bu | 2 | CFCl₂ | |
| 2-71 | Me | s-Bu | 2 | CFCl₂ | |
| 2-72 | Me | t-Bu | 2 | CFCl₂ | |
| 2-73 | Et | Me | 0 | CFCl₂ | |
| 2-74 | Et | Et | 0 | CFCl₂ | |
| 2-75 | Et | n-Pr | 0 | CFCl₂ | |
| 2-76 | Et | i-Pr | 0 | CFCl₂ | |
| 2-77 | Et | n-Bu | 0 | CFCl₂ | |
| 2-78 | Et | i-Bu | 0 | CFCl₂ | |
| 2-79 | Et | s-Bu | 0 | CFCl₂ | |
| 2-80 | Et | t-Bu | 0 | CFCl₂ | |
| 2-81 | Et | Me | 1 | CFCl₂ | |
| 2-82 | Et | Et | 1 | CFCl₂ | |
| 2-83 | Et | n-Pr | 1 | CFCl₂ | |
| 2-84 | Et | i-Pr | 1 | CFCl₂ | |
| 2-85 | Et | n-Bu | 1 | CFCl₂ | |
| 2-86 | Et | i-Bu | 1 | CFCl₂ | |
| 2-87 | Et | s-Bu | 1 | CFCl₂ | |
| 2-88 | Et | t-Bu | 1 | CFCl₂ | |
| 2-89 | Et | Me | 2 | CFCl₂ | |
| 2-90 | Et | Et | 2 | CFCl₂ | |
| 2-91 | Et | n-Pr | 2 | CFCl₂ | |
| 2-92 | Et | i-Pr | 2 | CFCl₂ | |
| 2-93 | Et | n-Bu | 2 | CFCl₂ | |
| 2-94 | Et | i-Bu | 2 | CFCl₂ | |
| 2-95 | Et | s-Bu | 2 | CFCl₂ | |
| 2-96 | Et | t-Bu | 2 | CFCl₂ | |
| 2-97 | Me | Me | 0 | CCl₃ | |
| 2-98 | Me | Et | 0 | CCl₃ | |
| 2-99 | Me | n-Pr | 0 | CCl₃ | |
| 2-100 | Me | i-Pr | 0 | CCl₃ | |
| 2-101 | Me | n-Bu | 0 | CCl₃ | |
| 2-102 | Me | i-Bu | 0 | CCl₃ | |
| 2-103 | Me | s-Bu | 0 | CCl₃ | |
| 2-104 | Me | t-Bu | 0 | CCl₃ | |
| 2-105 | Me | Me | 1 | CCl₃ | |
| 2-106 | Me | Et | 1 | CCl₃ | |
| 2-107 | Me | n-Pr | 1 | CCl₃ | |
| 2-108 | Me | i-Pr | 1 | CCl₃ | |
| 2-109 | Me | n-Bu | 1 | CCl₃ | |
| 2-110 | Me | i-Bu | 1 | CCl₃ | |
| 2-111 | Me | s-Bu | 1 | CCl₃ | |
| 2-112 | Me | t-Bu | 1 | CCl₃ | |
| 2-113 | Me | Me | 2 | CCl₃ | |
| 2-114 | Me | Et | 2 | CCl₃ | |
| 2-115 | Me | n-Pr | 2 | CCl₃ | |
| 2-116 | Me | i-Pr | 2 | CCl₃ | |
| 2-117 | Me | n-Bu | 2 | CCl₃ | |
| 2-118 | Me | i-Bu | 2 | CCl₃ | |
| 2-119 | Me | s-Bu | 2 | CCl₃ | |
| 2-120 | Me | t-Bu | 2 | CCl₃ | |
| 2-121 | Et | Me | 0 | CCl₃ | |
| 2-122 | Et | Et | 0 | CCl₃ | |
| 2-123 | Et | n-Pr | 0 | CCl₃ | |
| 2-124 | Et | i-Pr | 0 | CCl₃ | |
| 2-125 | Et | n-Bu | 0 | CCl₃ | |
| 2-126 | Et | i-Bu | 0 | CCl₃ | |
| 2-127 | Et | s-Bu | 0 | CCl₃ | |
| 2-128 | Et | t-Bu | 0 | CCl₃ | |
| 2-129 | Et | Me | 1 | CCl₃ | |
| 2-130 | Et | Et | 1 | CCl₃ | |
| 2-131 | Et | n-Pr | 1 | CCl₃ | |
| 2-132 | Et | i-Pr | 1 | CCl₃ | |
| 2-133 | Et | n-Bu | 1 | CCl₃ | |
| 2-134 | Et | i-Bu | 1 | CCl₃ | |
| 2-135 | Et | s-Bu | 1 | CCl₃ | |
| 2-136 | Et | t-Bu | 1 | CCl₃ | |
| 2-137 | Et | Me | 2 | CCl₃ | |
| 2-138 | Et | Et | 2 | CCl₃ | |
| 2-139 | Et | n-Pr | 2 | CCl₃ | |
| 2-140 | Et | i-Pr | 2 | CCl₃ | |
| 2-141 | Et | n-Bu | 2 | CCl₃ | |
| 2-142 | Et | i-Bu | 2 | CCl₃ | |
| 2-143 | Et | s-Bu | 2 | CCl₃ | |
| 2-144 | Et | t-Bu | 2 | CCl₃ | |
| 2-145 | Me | Me | 0 | C₂F₅ | |
| 2-146 | Me | Et | 0 | C₂F₅ | |
| 2-147 | Me | n-Pr | 0 | C₂F₅ | |
| 2-148 | Me | i-Pr | 0 | C₂F₅ | |
| 2-149 | Me | n-Bu | 0 | C₂F₅ | |
| 2-150 | Me | i-Bu | 0 | C₂F₅ | |
| 2-151 | Me | s-Bu | 0 | C₂F₅ | |
| 2-152 | Me | t-Bu | 0 | C₂F₅ | |
| 2-153 | Me | Me | 1 | C₂F₅ | |
| 2-154 | Me | Et | 1 | C₂F₅ | |
| 2-155 | Me | n-Pr | 1 | C₂F₅ | |
| 2-156 | Me | i-Pr | 1 | C₂F₅ | |
| 2-157 | Me | n-Bu | 1 | C₂F₅ | |
| 2-158 | Me | i-Bu | 1 | C₂F₅ | |
| 2-159 | Me | s-Bu | 1 | C₂F₅ | |
| 2-160 | Me | t-Bu | 1 | C₂F₅ | |
| 2-161 | Me | Me | 2 | C₂F₅ | |
| 2-162 | Me | Et | 2 | C₂F₅ | |
| 2-163 | Me | n-Pr | 2 | C₂F₅ | |
| 2-164 | Me | i-Pr | 2 | C₂F₅ | |
| 2-165 | Me | n-Bu | 2 | C₂F₅ | |
| 2-166 | Me | i-Bu | 2 | C₂F₅ | |
| 2-167 | Me | s-Bu | 2 | C₂F₅ | |
| 2-168 | Me | t-Bu | 2 | C₂F₅ | |
| 2-169 | Et | Me | 0 | C₂F₅ | |
| 2-170 | Et | Et | 0 | C₂F₅ | |
| 2-171 | Et | n-Pr | 0 | C₂F₅ | |
| 2-172 | Et | i-Pr | 0 | C₂F₅ | |
| 2-173 | Et | n-Bu | 0 | C₂F₅ | |
| 2-174 | Et | i-Bu | 0 | C₂F₅ | |
| 2-175 | Et | s-Bu | 0 | C₂F₅ | |
| 2-176 | Et | t-Bu | 0 | C₂F₅ | |
| 2-177 | Et | Me | 1 | C₂F₅ | |
| 2-178 | Et | Et | 1 | C₂F₅ | |
| 2-179 | Et | n-Pr | 1 | C₂F₅ | |
| 2-180 | Et | i-Pr | 1 | C₂F₅ | |
| 2-181 | Et | n-Bu | 1 | C₂F₅ | |
| 2-182 | Et | i-Bu | 1 | C₂F₅ | |
| 2-183 | Et | s-Bu | 1 | C₂F₅ | |
| 2-184 | Et | t-Bu | 1 | C₂F₅ | |
| 2-185 | Et | Me | 2 | C₂F₅ | |
| 2-186 | Et | Et | 2 | C₂F₅ | |
| 2-187 | Et | n-Pr | 2 | C₂F₅ | |
| 2-188 | Et | i-Pr | 2 | C₂F₅ | |
| 2-189 | Et | n-Bu | 2 | C₂F₅ | |
| 2-190 | Et | i-Bu | 2 | C₂F₅ | |
| 2-191 | Et | s-Bu | 2 | C₂F₅ | |
| 2-192 | Et | t-Bu | 2 | C₂F₅ | |
| 2-193 | Me | Me | 0 | CF(CF₃)₂ | |
| 2-194 | Me | Et | 0 | CF(CF₃)₂ | |
| 2-195 | Me | n-Pr | 0 | CF(CF₃)₂ | |
| 2-196 | Me | i-Pr | 0 | CF(CF₃)₂ | |
| 2-197 | Me | n-Bu | 0 | CF(CF₃)₂ | |
| 2-198 | Me | i-Bu | 0 | CF(CF₃)₂ | |
| 2-199 | Me | s-Bu | 0 | CF(CF₃)₂ | |
| 2-200 | Me | t-Bu | 0 | CF(CF₃)₂ | |
| 2-201 | Me | Me | 1 | CF(CF₃)₂ | |
| 2-202 | Me | Et | 1 | CF(CF₃)₂ | |
| 2-203 | Me | n-Pr | 1 | CF(CF₃)₂ | |
| 2-204 | Me | i-Pr | 1 | CF(CF₃)₂ | |
| 2-205 | Me | n-Bu | 1 | CF(CF₃)₂ | |
| 2-206 | Me | i-Bu | 1 | CF(CF₃)₂ | |
| 2-207 | Me | s-Bu | 1 | CF(CF₃)₂ | |
| 2-208 | Me | t-Bu | 1 | CF(CF₃)₂ | |
| 2-209 | Me | Me | 2 | CF(CF₃)₂ | |
| 2-210 | Me | Et | 2 | CF(CF₃)₂ | |
| 2-211 | Me | n-Pr | 2 | CF(CF₃)₂ | |
| 2-212 | Me | i-Pr | 2 | CF(CF₃)₂ | |
| 2-213 | Me | n-Bu | 2 | CF(CF₃)₂ | |
| 2-214 | Me | i-Bu | 2 | CF(CF₃)₂ | |
| 2-215 | Me | s-Bu | 2 | CF(CF₃)₂ | |
| 2-216 | Me | t-Bu | 2 | CF(CF₃)₂ | |
| 2-217 | Et | Me | 0 | CF(CF₃)₂ | |
| 2-218 | Et | Et | 0 | CF(CF₃)₂ | |
| 2-219 | Et | n-Pr | 0 | CF(CF₃)₂ | |
| 2-220 | Et | i-Pr | 0 | CF(CF₃)₂ | |
| 2-221 | Et | n-Bu | 0 | CF(CF₃)₂ | |
| 2-222 | Et | i-Bu | 0 | CF(CF₃)₂ | |
| 2-223 | Et | s-Bu | 0 | CF(CF₃)₂ | |
| 2-224 | Et | t-Bu | 0 | CF(CF₃)₂ | |
| 2-225 | Et | Me | 1 | CF(CF₃)₂ | |
| 2-226 | Et | Et | 1 | CF(CF₃)₂ | |
| 2-227 | Et | n-Pr | 1 | CF(CF₃)₂ | |
| 2-228 | Et | i-Pr | 1 | CF(CF₃)₂ | |
| 2-229 | Et | n-Bu | 1 | CF(CF₃)₂ | |
| 2-230 | Et | i-Bu | 1 | CF(CF₃)₂ | |
| 2-231 | Et | s-Bu | 1 | CF(CF₃)₂ | |
| 2-232 | Et | t-Bu | 1 | CF(CF₃)₂ | |
| 2-233 | Et | Me | 2 | CF(CF₃)₂ | |
| 2-234 | Et | Et | 2 | CF(CF₃)₂ | |
| 2-235 | Et | n-Pr | 2 | CF(CF₃)₂ | |
| 2-236 | Et | i-Pr | 2 | CF(CF₃)₂ | |
| 2-237 | Et | n-Bu | 2 | CF(CF₃)₂ | |
| 2-238 | Et | i-Bu | 2 | CF(CF₃)₂ | |
| 2-239 | Et | s-Bu | 2 | CF(CF₃)₂ | |
| 2-240 | Et | t-Bu | 2 | CF(CF₃)₂ | |
| 2-241 | Me | Me | 0 | CHF₂ | |
| 2-242 | Me | Et | 0 | CHF₂ | |
| 2-243 | Me | n-Pr | 0 | CHF₂ | |
| 2-244 | Me | i-Pr | 0 | CHF₂ | |
| 2-245 | Me | n-Bu | 0 | CHF₂ | |
| 2-246 | Me | i-Bu | 0 | CHF₂ | |
| 2-247 | Me | s-Bu | 0 | CHF₂ | |
| 2-248 | Me | t-Bu | 0 | CHF₂ | |
| 2-249 | Me | Me | 1 | CHF₂ | |
| 2-250 | Me | Et | 1 | CHF₂ | |
| 2-251 | Me | n-Pr | 1 | CHF₂ | |
| 2-252 | Me | i-Pr | 1 | CHF₂ | |
| 2-253 | Me | n-Bu | 1 | CHF₂ | |
| 2-254 | Me | i-Bu | 1 | CHF₂ | |
| 2-255 | Me | s-Bu | 1 | CHF₂ | |
| 2-256 | Me | t-Bu | 1 | CHF₂ | |
| 2-257 | Me | Me | 2 | CHF₂ | |
| 2-258 | Me | Et | 2 | CHF₂ | |
| 2-259 | Me | n-Pr | 2 | CHF₂ | |
| 2-260 | Me | i-Pr | 2 | CHF₂ | |
| 2-261 | Me | n-Bu | 2 | CHF₂ | |
| 2-262 | Me | i-Bu | 2 | CHF₂ | |
| 2-263 | Me | s-Bu | 2 | CHF₂ | |
| 2-264 | Me | t-Bu | 2 | CHF₂ | |
| 2-265 | Et | Me | 0 | CHF₂ | |
| 2-266 | Et | Et | 0 | CHF₂ | |
| 2-267 | Et | n-Pr | 0 | CHF₂ | |
| 2-268 | Et | i-Pr | 0 | CHF₂ | |
| 2-269 | Et | n-Bu | 0 | CHF₂ | |
| 2-270 | Et | i-Bu | 0 | CHF₂ | |
| 2-271 | Et | s-Bu | 0 | CHF₂ | |
| 2-272 | Et | t-Bu | 0 | CHF₂ | |
| 2-273 | Et | Me | 1 | CHF₂ | |
| 2-274 | Et | Et | 1 | CHF₂ | |
| 2-275 | Et | n-Pr | 1 | CHF₂ | |
| 2-276 | Et | i-Pr | 1 | CHF₂ | |
| 2-277 | Et | n-Bu | 1 | CHF₂ | |
| 2-278 | Et | i-Bu | 1 | CHF₂ | |
| 2-279 | Et | s-Bu | 1 | CHF₂ | |
| 2-280 | Et | t-Bu | 1 | CHF₂ | |
| 2-281 | Et | Me | 2 | CHF₂ | |
| 2-282 | Et | Et | 2 | CHF₂ | |
| 2-283 | Et | n-Pr | 2 | CHF₂ | |
| 2-284 | Et | i-Pr | 2 | CHF₂ | |
| 2-285 | Et | n-Bu | 2 | CHF₂ | |
| 2-286 | Et | i-Bu | 2 | CHF₂ | |
| 2-287 | Et | s-Bu | 2 | CHF₂ | |
| 2-288 | Et | t-Bu | 2 | CHF₂ | |

**Tabelle 3: Erfindungsgemäße Verbindungen der allgemeinen Formel (I), worin R² für Phenylthio, A für (CH₂), Z für (CH₂)₂, sowie R³ und R⁴ jeweils für Wasserstoff stehen**

| | | | | | |
|---|---|---|---|---|---|
| 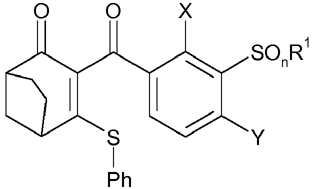 | | | | | |

| Nr. | X | R¹ | n | Y | Physikalische Daten: ¹H-NMR: δ [CDCl₃] |
|---|---|---|---|---|---|
| 3-1 | Me | Me | 0 | CF₂Cl | |
| 3-2 | Me | Et | 0 | CF₂Cl | |
| 3-3 | Me | n-Pr | 0 | CF₂Cl | |
| 3-4 | Me | i-Pr | 0 | CF₂Cl | |
| 3-5 | Me | n-Bu | 0 | CF₂Cl | |
| 3-6 | Me | i-Bu | 0 | CF₂Cl | |
| 3-7 | Me | s-Bu | 0 | CF₂Cl | |
| 3-8 | Me | t-Bu | 0 | CF₂Cl | |
| 3-9 | Me | Me | 1 | CF₂Cl | |
| 3-10 | Me | Et | 1 | CF₂Cl | |
| 3-11 | Me | n-Pr | 1 | CF₂Cl | |
| 3-12 | Me | i-Pr | 1 | CF₂Cl | |
| 3-13 | Me | n-Bu | 1 | CF₂Cl | |
| 3-14 | Me | i-Bu | 1 | CF₂Cl | |
| 3-15 | Me | s-Bu | 1 | CF₂Cl | |
| 3-16 | Me | t-Bu | 1 | CF₂Cl | |
| 3-17 | Me | Me | 2 | CF₂Cl | |
| 3-18 | Me | Et | 2 | CF₂Cl | |
| 3-19 | Me | n-Pr | 2 | CF₂Cl | |
| 3-20 | Me | i-Pr | 2 | CF₂Cl | |
| 3-21 | Me | n-Bu | 2 | CF₂Cl | |
| 3-22 | Me | i-Bu | 2 | CF₂Cl | |
| 3-23 | Me | s-Bu | 2 | CF₂Cl | |
| 3-24 | Me | t-Bu | 2 | CF₂Cl | |
| 3-25 | Et | Me | 0 | CF₂Cl | |
| 3-26 | Et | Et | 0 | CF₂Cl | |
| 3-27 | Et | n-Pr | 0 | CF₂Cl | |
| 3-28 | Et | i-Pr | 0 | CF₂Cl | |
| 3-29 | Et | n-Bu | 0 | CF₂Cl | |
| 3-30 | Et | i-Bu | 0 | CF₂Cl | |
| 3-31 | Et | s-Bu | 0 | CF₂Cl | |
| 3-32 | Et | t-Bu | 0 | CF₂Cl | |
| 3-33 | Et | Me | 1 | CF₂Cl | |
| 3-34 | Et | Et | 1 | CF₂Cl | |
| 3-35 | Et | n-Pr | 1 | CF₂Cl | |
| 3-36 | Et | i-Pr | 1 | CF₂Cl | |
| 3-37 | Et | n-Bu | 1 | CF₂Cl | |
| 3-38 | Et | i-Bu | 1 | CF₂Cl | |
| 3-39 | Et | s-Bu | 1 | CF₂Cl | |
| 3-40 | Et | t-Bu | 1 | CF₂Cl | |
| 3-41 | Et | Me | 2 | CF₂Cl | |
| 3-42 | Et | Et | 2 | CF₂Cl | |
| 3-43 | Et | n-Pr | 2 | CF₂Cl | |
| 3-44 | Et | i-Pr | 2 | CF₂Cl | |
| 3-45 | Et | n-Bu | 2 | CF₂Cl | |
| 3-46 | Et | i-Bu | 2 | CF₂Cl | |
| 3-47 | Et | s-Bu | 2 | CF₂Cl | |
| 3-48 | Et | t-Bu | 2 | CF₂Cl | |
| 3-49 | Me | Me | 0 | CFCl₂ | |
| 3-50 | Me | Et | 0 | CFCl₂ | |
| 3-51 | Me | n-Pr | 0 | CFCl₂ | |
| 3-52 | Me | i-Pr | 0 | CFCl₂ | |
| 3-53 | Me | n-Bu | 0 | CFCl₂ | |
| 3-54 | Me | i-Bu | 0 | CFCl₂ | |
| 3-55 | Me | s-Bu | 0 | CFCl₂ | |
| 3-56 | Me | t-Bu | 0 | CFCl₂ | |
| 3-57 | Me | Me | 1 | CFCl₂ | |
| 3-58 | Me | Et | 1 | CFCl₂ | |
| 3-59 | Me | n-Pr | 1 | CFCl₂ | |
| 3-60 | Me | i-Pr | 1 | CFCl₂ | |
| 3-61 | Me | n-Bu | 1 | CFCl₂ | |
| 3-62 | Me | i-Bu | 1 | CFCl₂ | |
| 3-63 | Me | s-Bu | 1 | CFCl₂ | |
| 3-64 | Me | t-Bu | 1 | CFCl₂ | |
| 3-65 | Me | Me | 2 | CFCl₂ | |
| 3-66 | Me | Et | 2 | CFCl₂ | |
| 3-67 | Me | n-Pr | 2 | CFCl₂ | |
| 3-68 | Me | i-Pr | 2 | CFCl₂ | |
| 3-69 | Me | n-Bu | 2 | CFCl₂ | |
| 3-70 | Me | i-Bu | 2 | CFCl₂ | |
| 3-71 | Me | s-Bu | 2 | CFCl₂ | |
| 3-72 | Me | t-Bu | 2 | CFCl₂ | |
| 3-73 | Et | Me | 0 | CFCl₂ | |
| 3-74 | Et | Et | 0 | CFCl₂ | |
| 3-75 | Et | n-Pr | 0 | CFCl₂ | |
| 3-76 | Et | i-Pr | 0 | CFCl₂ | |
| 3-77 | Et | n-Bu | 0 | CFCl₂ | |
| 3-78 | Et | i-Bu | 0 | CFCl₂ | |
| 3-79 | Et | s-Bu | 0 | CFCl₂ | |
| 3-80 | Et | t-Bu | 0 | CFCl₂ | |
| 3-81 | Et | Me | 1 | CFCl₂ | |
| 3-82 | Et | Et | 1 | CFCl₂ | |
| 3-83 | Et | n-Pr | 1 | CFCl₂ | |
| 3-84 | Et | i-Pr | 1 | CFCl₂ | |
| 3-85 | Et | n-Bu | 1 | CFCl₂ | |
| 3-86 | Et | i-Bu | 1 | CFCl₂ | |
| 3-87 | Et | s-Bu | 1 | CFCl₂ | |
| 3-88 | Et | t-Bu | 1 | CFCl₂ | |
| 3-89 | Et | Me | 2 | CFCl₂ | |
| 3-90 | Et | Et | 2 | CFCl₂ | |
| 3-91 | Et | n-Pr | 2 | CFCl₂ | |
| 3-92 | Et | i-Pr | 2 | CFCl₂ | |
| 3-93 | Et | n-Bu | 2 | CFCl₂ | |
| 3-94 | Et | i-Bu | 2 | CFCl₂ | |
| 3-95 | Et | s-Bu | 2 | CFCl₂ | |
| 3-96 | Et | t-Bu | 2 | CFCl₂ | |
| 3-97 | Me | Me | 0 | CCl₃ | |
| 3-98 | Me | Et | 0 | CCl₃ | |
| 3-99 | Me | n-Pr | 0 | CCl₃ | |
| 3-100 | Me | i-Pr | 0 | CCl₃ | |
| 3-101 | Me | n-Bu | 0 | CCl₃ | |
| 3-102 | Me | i-Bu | 0 | CCl₃ | |
| 3-103 | Me | s-Bu | 0 | CCl₃ | |
| 3-104 | Me | t-Bu | 0 | CCl₃ | |
| 3-105 | Me | Me | 1 | CCl₃ | |
| 3-106 | Me | Et | 1 | CCl₃ | |
| 3-107 | Me | n-Pr | 1 | CCl₃ | |
| 3-108 | Me | i-Pr | 1 | CCl₃ | |
| 3-109 | Me | n-Bu | 1 | CCl₃ | |
| 3-110 | Me | i-Bu | 1 | CCl₃ | |
| 3-111 | Me | s-Bu | 1 | CCl₃ | |
| 3-112 | Me | t-Bu | 1 | CCl₃ | |
| 3-113 | Me | Me | 2 | CCl₃ | |
| 3-114 | Me | Et | 2 | CCl₃ | |
| 3-115 | Me | n-Pr | 2 | CCl₃ | |
| 3-116 | Me | i-Pr | 2 | CCl₃ | |
| 3-117 | Me | n-Bu | 2 | CCl₃ | |
| 3-118 | Me | i-Bu | 2 | CCl₃ | |
| 3-119 | Me | s-Bu | 2 | CCl₃ | |
| 3-120 | Me | t-Bu | 2 | CCl₃ | |
| 3-121 | Et | Me | 0 | CCl₃ | |
| 3-122 | Et | Et | 0 | CCl₃ | |
| 3-123 | Et | n-Pr | 0 | CCl₃ | |
| 3-124 | Et | i-Pr | 0 | CCl₃ | |
| 3-125 | Et | n-Bu | 0 | CCl₃ | |
| 3-126 | Et | i-Bu | 0 | CCl₃ | |
| 3-127 | Et | s-Bu | 0 | CCl₃ | |
| 3-128 | Et | t-Bu | 0 | CCl₃ | |
| 3-129 | Et | Me | 1 | CCl₃ | |
| 3-130 | Et | Et | 1 | CCl₃ | |
| 3-131 | Et | n-Pr | 1 | CCl₃ | |
| 3-132 | Et | i-Pr | 1 | CCl₃ | |
| 3-133 | Et | n-Bu | 1 | CCl₃ | |
| 3-134 | Et | i-Bu | 1 | CCl₃ | |
| 3-135 | Et | s-Bu | 1 | CCl₃ | |
| 3-136 | Et | t-Bu | 1 | CCl₃ | |
| 3-137 | Et | Me | 2 | CCl₃ | |
| 3-138 | Et | Et | 2 | CCl₃ | |
| 3-139 | Et | n-Pr | 2 | CCl₃ | |
| 3-140 | Et | i-Pr | 2 | CCl₃ | |
| 3-141 | Et | n-Bu | 2 | CCl₃ | |
| 3-142 | Et | i-Bu | 2 | CCl₃ | |
| 3-143 | Et | s-Bu | 2 | CCl₃ | |
| 3-144 | Et | t-Bu | 2 | CCl₃ | |
| 3-145 | Me | Me | 0 | C₂F₅ | |
| 3-146 | Me | Et | 0 | C₂F₅ | |
| 3-147 | Me | n-Pr | 0 | C₂F₅ | |
| 3-148 | Me | i-Pr | 0 | C₂F₅ | |
| 3-149 | Me | n-Bu | 0 | C₂F₅ | |
| 3-150 | Me | i-Bu | 0 | C₂F₅ | |
| 3-151 | Me | s-Bu | 0 | C₂F₅ | |
| 3-152 | Me | t-Bu | 0 | C₂F₅ | |
| 3-153 | Me | Me | 1 | C₂F₅ | |
| 3-154 | Me | Et | 1 | C₂F₅ | |
| 3-155 | Me | n-Pr | 1 | C₂F₅ | |
| 3-156 | Me | i-Pr | 1 | C₂F₅ | |
| 3-157 | Me | n-Bu | 1 | C₂F₅ | |
| 3-158 | Me | i-Bu | 1 | C₂F₅ | |
| 3-159 | Me | s-Bu | 1 | C₂F₅ | |
| 3-160 | Me | t-Bu | 1 | C₂F₅ | |
| 3-161 | Me | Me | 2 | C₂F₅ | |
| 3-162 | Me | Et | 2 | C₂F₅ | |
| 3-163 | Me | n-Pr | 2 | C₂F₅ | |
| 3-164 | Me | i-Pr | 2 | C₂F₅ | |
| 3-165 | Me | n-Bu | 2 | C₂F₅ | |
| 3-166 | Me | i-Bu | 2 | C₂F₅ | |
| 3-167 | Me | s-Bu | 2 | C₂F₅ | |
| 3-168 | Me | t-Bu | 2 | C₂F₅ | |
| 3-169 | Et | Me | 0 | C₂F₅ | |
| 3-170 | Et | Et | 0 | C₂F₅ | |
| 3-171 | Et | n-Pr | 0 | C₂F₅ | |
| 3-172 | Et | i-Pr | 0 | C₂F₅ | |
| 3-173 | Et | n-Bu | 0 | C₂F₅ | |
| 3-174 | Et | i-Bu | 0 | C₂F₅ | |
| 3-175 | Et | s-Bu | 0 | C₂F₅ | |
| 3-176 | Et | t-Bu | 0 | C₂F₅ | |
| 3-177 | Et | Me | 1 | C₂F₅ | |
| 3-178 | Et | Et | 1 | C₂F₅ | |
| 3-179 | Et | n-Pr | 1 | C₂F₅ | |
| 3-180 | Et | i-Pr | 1 | C₂F₅ | |
| 3-181 | Et | n-Bu | 1 | C₂F₅ | |
| 3-182 | Et | i-Bu | 1 | C₂F₅ | |
| 3-183 | Et | s-Bu | 1 | C₂F₅ | |
| 3-184 | Et | t-Bu | 1 | C₂F₅ | |
| 3-185 | Et | Me | 2 | C₂F₅ | |
| 3-186 | Et | Et | 2 | C₂F₅ | |
| 3-187 | Et | n-Pr | 2 | C₂F₅ | |
| 3-188 | Et | i-Pr | 2 | C₂F₅ | |
| 3-189 | Et | n-Bu | 2 | C₂F₅ | |
| 3-190 | Et | i-Bu | 2 | C₂F₅ | |
| 3-191 | Et | s-Bu | 2 | C₂F₅ | |
| 3-192 | Et | t-Bu | 2 | C₂F₅ | |
| 3-193 | Me | Me | 0 | CF(CF₃)₂ | |
| 3-194 | Me | Et | 0 | CF(CF₃)₂ | |
| 3-195 | Me | n-Pr | 0 | CF(CF₃)₂ | |
| 3-196 | Me | i-Pr | 0 | CF(CF₃)₂ | |
| 3-197 | Me | n-Bu | 0 | CF(CF₃)₂ | |
| 3-198 | Me | i-Bu | 0 | CF(CF₃)₂ | |
| 3-199 | Me | s-Bu | 0 | CF(CF₃)₂ | |
| 3-200 | Me | t-Bu | 0 | CF(CF₃)₂ | |
| 3-201 | Me | Me | 1 | CF(CF₃)₂ | |
| 3-202 | Me | Et | 1 | CF(CF₃)₂ | |
| 3-203 | Me | n-Pr | 1 | CF(CF₃)₂ | |
| 3-204 | Me | i-Pr | 1 | CF(CF₃)₂ | |
| 3-205 | Me | n-Bu | 1 | CF(CF₃)₂ | |
| 3-206 | Me | i-Bu | 1 | CF(CF₃)₂ | |
| 3-207 | Me | s-Bu | 1 | CF(CF₃)₂ | |
| 3-208 | Me | t-Bu | 1 | CF(CF₃)₂ | |
| 3-209 | Me | Me | 2 | CF(CF₃)₂ | |
| 3-210 | Me | Et | 2 | CF(CF₃)₂ | |
| 3-211 | Me | n-Pr | 2 | CF(CF₃)₂ | |
| 3-212 | Me | i-Pr | 2 | CF(CF₃)₂ | |
| 3-213 | Me | n-Bu | 2 | CF(CF₃)₂ | |
| 3-214 | Me | i-Bu | 2 | CF(CF₃)₂ | |
| 3-215 | Me | s-Bu | 2 | CF(CF₃)₂ | |
| 3-216 | Me | t-Bu | 2 | CF(CF₃)₂ | |
| 3-217 | Et | Me | 0 | CF(CF₃)₂ | |
| 3-218 | Et | Et | 0 | CF(CF₃)₂ | |
| 3-219 | Et | n-Pr | 0 | CF(CF₃)₂ | |
| 3-220 | Et | i-Pr | 0 | CF(CF₃)₂ | |
| 3-221 | Et | n-Bu | 0 | CF(CF₃)₂ | |
| 3-222 | Et | i-Bu | 0 | CF(CF₃)₂ | |
| 3-223 | Et | s-Bu | 0 | CF(CF₃)₂ | |
| 3-224 | Et | t-Bu | 0 | CF(CF₃)₂ | |
| 3-225 | Et | Me | 1 | CF(CF₃)₂ | |
| 3-226 | Et | Et | 1 | CF(CF₃)₂ | |
| 3-227 | Et | n-Pr | 1 | CF(CF₃)₂ | |
| 3-228 | Et | i-Pr | 1 | CF(CF₃)₂ | |
| 3-229 | Et | n-Bu | 1 | CF(CF₃)₂ | |
| 3-230 | Et | i-Bu | 1 | CF(CF₃)₂ | |
| 3-231 | Et | s-Bu | 1 | CF(CF₃)₂ | |
| 3-232 | Et | t-Bu | 1 | CF(CF₃)₂ | |
| 3-233 | Et | Me | 2 | CF(CF₃)₂ | |
| 3-234 | Et | Et | 2 | CF(CF₃)₂ | |
| 3-235 | Et | n-Pr | 2 | CF(CF₃)₂ | |
| 3-236 | Et | i-Pr | 2 | CF(CF₃)₂ | |
| 3-237 | Et | n-Bu | 2 | CF(CF₃)₂ | |
| 3-238 | Et | i-Bu | 2 | CF(CF₃)₂ | |
| 3-239 | Et | s-Bu | 2 | CF(CF₃)₂ | |
| 3-240 | Et | t-Bu | 2 | CF(CF₃)₂ | |
| 3-241 | Me | Me | 0 | CHF₂ | |
| 3-242 | Me | Et | 0 | CHF₂ | |
| 3-243 | Me | n-Pr | 0 | CHF₂ | |
| 3-244 | Me | i-Pr | 0 | CHF₂ | |
| 3-245 | Me | n-Bu | 0 | CHF₂ | |
| 3-246 | Me | i-Bu | 0 | CHF₂ | |
| 3-247 | Me | s-Bu | 0 | CHF₂ | |
| 3-248 | Me | t-Bu | 0 | CHF₂ | |
| 3-249 | Me | Me | 1 | CHF₂ | |
| 3-250 | Me | Et | 1 | CHF₂ | |
| 3-251 | Me | n-Pr | 1 | CHF₂ | |
| 3-252 | Me | i-Pr | 1 | CHF₂ | |
| 3-253 | Me | n-Bu | 1 | CHF₂ | |
| 3-254 | Me | i-Bu | 1 | CHF₂ | |
| 3-255 | Me | s-Bu | 1 | CHF₂ | |
| 3-256 | Me | t-Bu | 1 | CHF₂ | |
| 3-257 | Me | Me | 2 | CHF₂ | |
| 3-258 | Me | Et | 2 | CHF₂ | |
| 3-259 | Me | n-Pr | 2 | CHF₂ | |
| 3-260 | Me | i-Pr | 2 | CHF₂ | |
| 3-261 | Me | n-Bu | 2 | CHF₂ | |
| 3-262 | Me | i-Bu | 2 | CHF₂ | |
| 3-263 | Me | s-Bu | 2 | CHF₂ | |
| 3-264 | Me | t-Bu | 2 | CHF₂ | |
| 3-265 | Et | Me | 0 | CHF₂ | |
| 3-266 | Et | Et | 0 | CHF₂ | |
| 3-267 | Et | n-Pr | 0 | CHF₂ | |
| 3-268 | Et | i-Pr | 0 | CHF₂ | |
| 3-269 | Et | n-Bu | 0 | CHF₂ | |
| 3-270 | Et | i-Bu | 0 | CHF₂ | |
| 3-271 | Et | s-Bu | 0 | CHF₂ | |
| 3-272 | Et | t-Bu | 0 | CHF₂ | |
| 3-273 | Et | Me | 1 | CHF₂ | |
| 3-274 | Et | Et | 1 | CHF₂ | |
| 3-275 | Et | n-Pr | 1 | CHF₂ | |
| 3-276 | Et | i-Pr | 1 | CHF₂ | |
| 3-277 | Et | n-Bu | 1 | CHF₂ | |
| 3-278 | Et | i-Bu | 1 | CHF₂ | |
| 3-279 | Et | s-Bu | 1 | CHF₂ | |
| 3-280 | Et | t-Bu | 1 | CHF₂ | |
| 3-281 | Et | Me | 2 | CHF₂ | |
| 3-282 | Et | Et | 2 | CHF₂ | |
| 3-283 | Et | n-Pr | 2 | CHF₂ | |
| 3-284 | Et | i-Pr | 2 | CHF₂ | |
| 3-285 | Et | n-Bu | 2 | CHF₂ | |
| 3-286 | Et | i-Bu | 2 | CHF₂ | |
| 3-287 | Et | s-Bu | 2 | CHF₂ | |
| 3-288 | Et | t-Bu | 2 | CHF₂ | |

**Tabelle 4: Erfindungsgemäße Verbindungen der allgemeinen Formel (I), worin R² für Phenylthio, A für Sauerstoff, Z für (CH₂)₂, R³ für Methyl sowie R⁴ für Wasserstoff stehen**

| | | | | | |
|---|---|---|---|---|---|
| 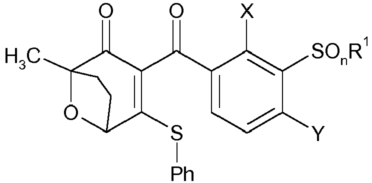 | | | | | |

| Nr. | X | R¹ | n | Y | Physikalische Daten: ¹H-NMR: δ [CDCl₃] |
|---|---|---|---|---|---|
| 4-1 | Me | Me | 0 | CF₂Cl | |
| 4-2 | Me | Et | 0 | CF₂Cl | |
| 4-3 | Me | n-Pr | 0 | CF₂Cl | |
| 4-4 | Me | i-Pr | 0 | CF₂Cl | |
| 4-5 | Me | n-Bu | 0 | CF₂Cl | |
| 4-6 | Me | i-Bu | 0 | CF₂Cl | |
| 4-7 | Me | s-Bu | 0 | CF₂Cl | |
| 4-8 | Me | t-Bu | 0 | CF₂Cl | |
| 4-9 | Me | Me | 1 | CF₂Cl | |
| 4-10 | Me | Et | 1 | CF₂Cl | |
| 4-11 | Me | n-Pr | 1 | CF₂Cl | |
| 4-12 | Me | i-Pr | 1 | CF₂Cl | |
| 4-13 | Me | n-Bu | 1 | CF₂Cl | |
| 4-14 | Me | i-Bu | 1 | CF₂Cl | |
| 4-15 | Me | s-Bu | 1 | CF₂Cl | |
| 4-16 | Me | t-Bu | 1 | CF₂Cl | |
| 4-17 | Me | Me | 2 | CF₂Cl | |
| 4-18 | Me | Et | 2 | CF₂Cl | |
| 4-19 | Me | n-Pr | 2 | CF₂Cl | |
| 4-20 | Me | i-Pr | 2 | CF₂Cl | |
| 4-21 | Me | n-Bu | 2 | CF₂Cl | |
| 4-22 | Me | i-Bu | 2 | CF₂Cl | |
| 4-23 | Me | s-Bu | 2 | CF₂Cl | |
| 4-24 | Me | t-Bu | 2 | CF₂Cl | |
| 4-25 | Et | Me | 0 | CF₂Cl | |
| 4-26 | Et | Et | 0 | CF₂Cl | |
| 4-27 | Et | n-Pr | 0 | CF₂Cl | |
| 4-28 | Et | i-Pr | 0 | CF₂Cl | |
| 4-29 | Et | n-Bu | 0 | CF₂Cl | |
| 4-30 | Et | i-Bu | 0 | CF₂Cl | |
| 4-31 | Et | s-Bu | 0 | CF₂Cl | |
| 4-32 | Et | t-Bu | 0 | CF₂Cl | |
| 4-33 | Et | Me | 1 | CF₂Cl | |
| 4-34 | Et | Et | 1 | CF₂Cl | |
| 4-35 | Et | n-Pr | 1 | CF₂Cl | |
| 4-36 | Et | i-Pr | 1 | CF₂Cl | |
| 4-37 | Et | n-Bu | 1 | CF₂Cl | |
| 4-38 | Et | i-Bu | 1 | CF₂Cl | |
| 4-39 | Et | s-Bu | 1 | CF₂Cl | |
| 4-40 | Et | t-Bu | 1 | CF₂Cl | |
| 4-41 | Et | Me | 2 | CF₂Cl | |
| 4-42 | Et | Et | 2 | CF₂Cl | |
| 4-43 | Et | n-Pr | 2 | CF₂Cl | |
| 4-44 | Et | i-Pr | 2 | CF₂Cl | |
| 4-45 | Et | n-Bu | 2 | CF₂Cl | |
| 4-46 | Et | i-Bu | 2 | CF₂Cl | |
| 4-47 | Et | s-Bu | 2 | CF₂Cl | |
| 4-48 | Et | t-Bu | 2 | CF₂Cl | |
| 4-49 | Me | Me | 0 | CFCl₂ | |
| 4-50 | Me | Et | 0 | CFCl₂ | |
| 4-51 | Me | n-Pr | 0 | CFCl₂ | |
| 4-52 | Me | i-Pr | 0 | CFCl₂ | |
| 4-53 | Me | n-Bu | 0 | CFCl₂ | |
| 4-54 | Me | i-Bu | 0 | CFCl₂ | |
| 4-55 | Me | s-Bu | 0 | CFCl₂ | |
| 4-56 | Me | t-Bu | 0 | CFCl₂ | |
| 4-57 | Me | Me | 1 | CFCl₂ | |
| 4-58 | Me | Et | 1 | CFCl₂ | |
| 4-59 | Me | n-Pr | 1 | CFCl₂ | |
| 4-60 | Me | i-Pr | 1 | CFCl₂ | |
| 4-61 | Me | n-Bu | 1 | CFCl₂ | |
| 4-62 | Me | i-Bu | 1 | CFCl₂ | |
| 4-63 | Me | s-Bu | 1 | CFCl₂ | |
| 4-64 | Me | t-Bu | 1 | CFCl₂ | |
| 4-65 | Me | Me | 2 | CFCl₂ | |
| 4-66 | Me | Et | 2 | CFCl₂ | |
| 4-67 | Me | n-Pr | 2 | CFCl₂ | |
| 4-68 | Me | i-Pr | 2 | CFCl₂ | |
| 4-69 | Me | n-Bu | 2 | CFCl₂ | |
| 4-70 | Me | i-Bu | 2 | CFCl₂ | |
| 4-71 | Me | s-Bu | 2 | CFCl₂ | |
| 4-72 | Me | t-Bu | 2 | CFCl₂ | |
| 4-73 | Et | Me | 0 | CFCl₂ | |
| 4-74 | Et | Et | 0 | CFCl₂ | |
| 4-75 | Et | n-Pr | 0 | CFCl₂ | |
| 4-76 | Et | i-Pr | 0 | CFCl₂ | |
| 4-77 | Et | n-Bu | 0 | CFCl₂ | |
| 4-78 | Et | i-Bu | 0 | CFCl₂ | |
| 4-79 | Et | s-Bu | 0 | CFCl₂ | |
| 4-80 | Et | t-Bu | 0 | CFCl₂ | |
| 4-81 | Et | Me | 1 | CFCl₂ | |
| 4-82 | Et | Et | 1 | CFCl₂ | |
| 4-83 | Et | n-Pr | 1 | CFCl₂ | |
| 4-84 | Et | i-Pr | 1 | CFCl₂ | |
| 4-85 | Et | n-Bu | 1 | CFCl₂ | |
| 4-86 | Et | i-Bu | 1 | CFCl₂ | |
| 4-87 | Et | s-Bu | 1 | CFCl₂ | |
| 4-88 | Et | t-Bu | 1 | CFCl₂ | |
| 4-89 | Et | Me | 2 | CFCl₂ | |
| 4-90 | Et | Et | 2 | CFCl₂ | |
| 4-91 | Et | n-Pr | 2 | CFCl₂ | |
| 4-92 | Et | i-Pr | 2 | CFCl₂ | |
| 4-93 | Et | n-Bu | 2 | CFCl₂ | |
| 4-94 | Et | i-Bu | 2 | CFCl₂ | |
| 4-95 | Et | s-Bu | 2 | CFCl₂ | |
| 4-96 | Et | t-Bu | 2 | CFCl₂ | |
| 4-97 | Me | Me | 0 | CCl₃ | |
| 4-98 | Me | Et | 0 | CCl₃ | |
| 4-99 | Me | n-Pr | 0 | CCl₃ | |
| 4-100 | Me | i-Pr | 0 | CCl₃ | |
| 4-101 | Me | n-Bu | 0 | CCl₃ | |
| 4-102 | Me | i-Bu | 0 | CCl₃ | |
| 4-103 | Me | s-Bu | 0 | CCl₃ | |
| 4-104 | Me | t-Bu | 0 | CCl₃ | |
| 4-105 | Me | Me | 1 | CCl₃ | |
| 4-106 | Me | Et | 1 | CCl₃ | |
| 4-107 | Me | n-Pr | 1 | CCl₃ | |
| 4-108 | Me | i-Pr | 1 | CCl₃ | |
| 4-109 | Me | n-Bu | 1 | CCl₃ | |
| 4-110 | Me | i-Bu | 1 | CCl₃ | |
| 4-111 | Me | s-Bu | 1 | CCl₃ | |
| 4-112 | Me | t-Bu | 1 | CCl₃ | |
| 4-113 | Me | Me | 2 | CCl₃ | |
| 4-114 | Me | Et | 2 | CCl₃ | |
| 4-115 | Me | n-Pr | 2 | CCl₃ | |
| 4-116 | Me | i-Pr | 2 | CCl₃ | |
| 4-117 | Me | n-Bu | 2 | CCl₃ | |
| 4-118 | Me | i-Bu | 2 | CCl₃ | |
| 4-119 | Me | s-Bu | 2 | CCl₃ | |
| 4-120 | Me | t-Bu | 2 | CCl₃ | |
| 4-121 | Et | Me | 0 | CCl₃ | |
| 4-122 | Et | Et | 0 | CCl₃ | |
| 4-123 | Et | n-Pr | 0 | CCl₃ | |
| 4-124 | Et | i-Pr | 0 | CCl₃ | |
| 4-125 | Et | n-Bu | 0 | CCl₃ | |
| 4-126 | Et | i-Bu | 0 | CCl₃ | |
| 4-127 | Et | s-Bu | 0 | CCl₃ | |
| 4-128 | Et | t-Bu | 0 | CCl₃ | |
| 4-129 | Et | Me | 1 | CCl₃ | |
| 4-130 | Et | Et | 1 | CCl₃ | |
| 4-131 | Et | n-Pr | 1 | CCl₃ | |
| 4-132 | Et | i-Pr | 1 | CCl₃ | |
| 4-133 | Et | n-Bu | 1 | CCl₃ | |
| 4-134 | Et | i-Bu | 1 | CCl₃ | |
| 4-135 | Et | s-Bu | 1 | CCl₃ | |
| 4-136 | Et | t-Bu | 1 | CCl₃ | |
| 4-137 | Et | Me | 2 | CCl₃ | |
| 4-138 | Et | Et | 2 | CCl₃ | |
| 4-139 | Et | n-Pr | 2 | CCl₃ | |
| 4-140 | Et | i-Pr | 2 | CCl₃ | |
| 4-141 | Et | n-Bu | 2 | CCl₃ | |
| 4-142 | Et | i-Bu | 2 | CCl₃ | |
| 4-143 | Et | s-Bu | 2 | CCl₃ | |
| 4-144 | Et | t-Bu | 2 | CCl₃ | |
| 4-145 | Me | Me | 0 | C₂F₅ | |
| 4-146 | Me | Et | 0 | C₂F₅ | |
| 4-147 | Me | n-Pr | 0 | C₂F₅ | |
| 4-148 | Me | i-Pr | 0 | C₂F₅ | |
| 4-149 | Me | n-Bu | 0 | C₂F₅ | |
| 4-150 | Me | i-Bu | 0 | C₂F₅ | |
| 4-151 | Me | s-Bu | 0 | C₂F₅ | |
| 4-152 | Me | t-Bu | 0 | C₂F₅ | |
| 4-153 | Me | Me | 1 | C₂F₅ | |
| 4-154 | Me | Et | 1 | C₂F₅ | |
| 4-155 | Me | n-Pr | 1 | C₂F₅ | |
| 4-156 | Me | i-Pr | 1 | C₂F₅ | |
| 4-157 | Me | n-Bu | 1 | C₂F₅ | |
| 4-158 | Me | i-Bu | 1 | C₂F₅ | |
| 4-159 | Me | s-Bu | 1 | C₂F₅ | |
| 4-160 | Me | t-Bu | 1 | C₂F₅ | |
| 4-161 | Me | Me | 2 | C₂F₅ | |
| 4-162 | Me | Et | 2 | C₂F₅ | |
| 4-163 | Me | n-Pr | 2 | C₂F₅ | |
| 4-164 | Me | i-Pr | 2 | C₂F₅ | |
| 4-165 | Me | n-Bu | 2 | C₂F₅ | |
| 4-166 | Me | i-Bu | 2 | C₂F₅ | |
| 4-167 | Me | s-Bu | 2 | C₂F₅ | |
| 4-168 | Me | t-Bu | 2 | C₂F₅ | |
| 4-169 | Et | Me | 0 | C₂F₅ | |
| 4-170 | Et | Et | 0 | C₂F₅ | |
| 4-171 | Et | n-Pr | 0 | C₂F₅ | |
| 4-172 | Et | i-Pr | 0 | C₂F₅ | |
| 4-173 | Et | n-Bu | 0 | C₂F₅ | |
| 4-174 | Et | i-Bu | 0 | C₂F₅ | |
| 4-175 | Et | s-Bu | 0 | C₂F₅ | |
| 4-176 | Et | t-Bu | 0 | C₂F₅ | |
| 4-177 | Et | Me | 1 | C₂F₅ | |
| 4-178 | Et | Et | 1 | C₂F₅ | |
| 4-179 | Et | n-Pr | 1 | C₂F₅ | |
| 4-180 | Et | i-Pr | 1 | C₂F₅ | |
| 4-181 | Et | n-Bu | 1 | C₂F₅ | |
| 4-182 | Et | i-Bu | 1 | C₂F₅ | |
| 4-183 | Et | s-Bu | 1 | C₂F₅ | |
| 4-184 | Et | t-Bu | 1 | C₂F₅ | |
| 4-185 | Et | Me | 2 | C₂F₅ | |
| 4-186 | Et | Et | 2 | C₂F₅ | |
| 4-187 | Et | n-Pr | 2 | C₂F₅ | |
| 4-188 | Et | i-Pr | 2 | C₂F₅ | |
| 4-189 | Et | n-Bu | 2 | C₂F₅ | |
| 4-190 | Et | i-Bu | 2 | C₂F₅ | |
| 4-191 | Et | s-Bu | 2 | C₂F₅ | |
| 4-192 | Et | t-Bu | 2 | C₂F₅ | |
| 4-193 | Me | Me | 0 | CF(CF₃)₂ | |
| 4-194 | Me | Et | 0 | CF(CF₃)₂ | |
| 4-195 | Me | n-Pr | 0 | CF(CF₃)₂ | |
| 4-196 | Me | i-Pr | 0 | CF(CF₃)₂ | |
| 4-197 | Me | n-Bu | 0 | CF(CF₃)₂ | |
| 4-198 | Me | i-Bu | 0 | CF(CF₃)₂ | |
| 4-199 | Me | s-Bu | 0 | CF(CF₃)₂ | |
| 4-200 | Me | t-Bu | 0 | CF(CF₃)₂ | |
| 4-201 | Me | Me | 1 | CF(CF₃)₂ | |
| 4-202 | Me | Et | 1 | CF(CF₃)₂ | |
| 4-203 | Me | n-Pr | 1 | CF(CF₃)₂ | |
| 4-204 | Me | i-Pr | 1 | CF(CF₃)₂ | |
| 4-205 | Me | n-Bu | 1 | CF(CF₃)₂ | |
| 4-206 | Me | i-Bu | 1 | CF(CF₃)₂ | |
| 4-207 | Me | s-Bu | 1 | CF(CF₃)₂ | |
| 4-208 | Me | t-Bu | 1 | CF(CF₃)₂ | |
| 4-209 | Me | Me | 2 | CF(CF₃)₂ | |
| 4-210 | Me | Et | 2 | CF(CF₃)₂ | |
| 4-211 | Me | n-Pr | 2 | CF(CF₃)₂ | |
| 4-212 | Me | i-Pr | 2 | CF(CF₃)₂ | |
| 4-213 | Me | n-Bu | 2 | CF(CF₃)₂ | |
| 4-214 | Me | i-Bu | 2 | CF(CF₃)₂ | |
| 4-215 | Me | s-Bu | 2 | CF(CF₃)₂ | |
| 4-216 | Me | t-Bu | 2 | CF(CF₃)₂ | |
| 4-217 | Et | Me | 0 | CF(CF₃)₂ | |
| 4-218 | Et | Et | 0 | CF(CF₃)₂ | |
| 4-219 | Et | n-Pr | 0 | CF(CF₃)₂ | |
| 4-220 | Et | i-Pr | 0 | CF(CF₃)₂ | |
| 4-221 | Et | n-Bu | 0 | CF(CF₃)₂ | |
| 4-222 | Et | i-Bu | 0 | CF(CF₃)₂ | |
| 4-223 | Et | s-Bu | 0 | CF(CF₃)₂ | |
| 4-224 | Et | t-Bu | 0 | CF(CF₃)₂ | |
| 4-225 | Et | Me | 1 | CF(CF₃)₂ | |
| 4-226 | Et | Et | 1 | CF(CF₃)₂ | |
| 4-227 | Et | n-Pr | 1 | CF(CF₃)₂ | |
| 4-228 | Et | i-Pr | 1 | CF(CF₃)₂ | |
| 4-229 | Et | n-Bu | 1 | CF(CF₃)₂ | |
| 4-230 | Et | i-Bu | 1 | CF(CF₃)₂ | |
| 4-231 | Et | s-Bu | 1 | CF(CF₃)₂ | |
| 4-232 | Et | t-Bu | 1 | CF(CF₃)₂ | |
| 4-233 | Et | Me | 2 | CF(CF₃)₂ | |
| 4-234 | Et | Et | 2 | CF(CF₃)₂ | |
| 4-235 | Et | n-Pr | 2 | CF(CF₃)₂ | |
| 4-236 | Et | i-Pr | 2 | CF(CF₃)₂ | |
| 4-237 | Et | n-Bu | 2 | CF(CF₃)₂ | |
| 4-238 | Et | i-Bu | 2 | CF(CF₃)₂ | |
| 4-239 | Et | s-Bu | 2 | CF(CF₃)₂ | |
| 4-240 | Et | t-Bu | 2 | CF(CF₃)₂ | |
| 4-241 | Me | Me | 0 | CHF₂ | |
| 4-242 | Me | Et | 0 | CHF₂ | |
| 4-243 | Me | n-Pr | 0 | CHF₂ | |
| 4-244 | Me | i-Pr | 0 | CHF₂ | |
| 4-245 | Me | n-Bu | 0 | CHF₂ | |
| 4-246 | Me | i-Bu | 0 | CHF₂ | |
| 4-247 | Me | s-Bu | 0 | CHF₂ | |
| 4-248 | Me | t-Bu | 0 | CHF₂ | |
| 4-249 | Me | Me | 1 | CHF₂ | |
| 4-250 | Me | Et | 1 | CHF₂ | |
| 4-251 | Me | n-Pr | 1 | CHF₂ | |
| 4-252 | Me | i-Pr | 1 | CHF₂ | |
| 4-253 | Me | n-Bu | 1 | CHF₂ | |
| 4-254 | Me | i-Bu | 1 | CHF₂ | |
| 4-255 | Me | s-Bu | 1 | CHF₂ | |
| 4-256 | Me | t-Bu | 1 | CHF₂ | |
| 4-257 | Me | Me | 2 | CHF₂ | |
| 4-258 | Me | Et | 2 | CHF₂ | |
| 4-259 | Me | n-Pr | 2 | CHF₂ | |
| 4-260 | Me | i-Pr | 2 | CHF₂ | |
| 4-261 | Me | n-Bu | 2 | CHF₂ | |
| 4-262 | Me | i-Bu | 2 | CHF₂ | |
| 4-263 | Me | s-Bu | 2 | CHF₂ | |
| 4-264 | Me | t-Bu | 2 | CHF₂ | |
| 4-265 | Et | Me | 0 | CHF₂ | |
| 4-266 | Et | Et | 0 | CHF₂ | |
| 4-267 | Et | n-Pr | 0 | CHF₂ | |
| 4-268 | Et | i-Pr | 0 | CHF₂ | |
| 4-269 | Et | n-Bu | 0 | CHF₂ | |
| 4-270 | Et | i-Bu | 0 | CHF₂ | |
| 4-271 | Et | s-Bu | 0 | CHF₂ | |
| 4-272 | Et | t-Bu | 0 | CHF₂ | |
| 4-273 | Et | Me | 1 | CHF₂ | |
| 4-274 | Et | Et | 1 | CHF₂ | |
| 4-275 | Et | n-Pr | 1 | CHF₂ | |
| 4-276 | Et | i-Pr | 1 | CHF₂ | |
| 4-277 | Et | n-Bu | 1 | CHF₂ | |
| 4-278 | Et | i-Bu | 1 | CHF₂ | |
| 4-279 | Et | s-Bu | 1 | CHF₂ | |
| 4-280 | Et | t-Bu | 1 | CHF₂ | |
| 4-281 | Et | Me | 2 | CHF₂ | |
| 4-282 | Et | Et | 2 | CHF₂ | |
| 4-283 | Et | n-Pr | 2 | CHF₂ | |
| 4-284 | Et | i-Pr | 2 | CHF₂ | |
| 4-285 | Et | n-Bu | 2 | CHF₂ | |
| 4-286 | Et | i-Bu | 2 | CHF₂ | |
| 4-287 | Et | s-Bu | 2 | CHF₂ | |
| 4-288 | Et | t-Bu | 2 | CHF₂ | |

**Tabelle 5: Erfindungsgemäße Verbindungen der allgemeinen Formel (I), worin R² für Phenylthio, A für Sauerstoff, Z für (CH₂)₂, R³ für Wasserstoff sowie R⁴ für Methyl stehen**

| | | | | | |
|---|---|---|---|---|---|
| 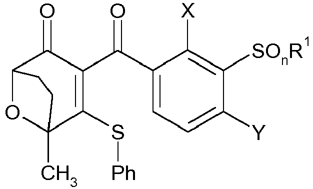 | | | | | |

| Nr. | X | R¹ | n | Y | Physikalische Daten: ¹H-NMR: δ [CDCl₃] |
|---|---|---|---|---|---|
| 5-1 | Me | Me | 0 | CF₂Cl | |
| 5-2 | Me | Et | 0 | CF₂Cl | |
| 5-3 | Me | n-Pr | 0 | CF₂Cl | |
| 5-4 | Me | i-Pr | 0 | CF₂Cl | |
| 5-5 | Me | n-Bu | 0 | CF₂Cl | |
| 5-6 | Me | i-Bu | 0 | CF₂Cl | |
| 5-7 | Me | s-Bu | 0 | CF₂Cl | |
| 5-8 | Me | t-Bu | 0 | CF₂Cl | |
| 5-9 | Me | Me | 1 | CF₂Cl | |
| 5-10 | Me | Et | 1 | CF₂Cl | |
| 5-11 | Me | n-Pr | 1 | CF₂Cl | |
| 5-12 | Me | i-Pr | 1 | CF₂Cl | |
| 5-13 | Me | n-Bu | 1 | CF₂Cl | |
| 5-14 | Me | i-Bu | 1 | CF₂Cl | |
| 5-15 | Me | s-Bu | 1 | CF₂Cl | |
| 5-16 | Me | t-Bu | 1 | CF₂Cl | |
| 5-17 | Me | Me | 2 | CF₂Cl | |
| 5-18 | Me | Et | 2 | CF₂Cl | |
| 5-19 | Me | n-Pr | 2 | CF₂Cl | |
| 5-20 | Me | i-Pr | 2 | CF₂Cl | |
| 5-21 | Me | n-Bu | 2 | CF₂Cl | |
| 5-22 | Me | i-Bu | 2 | CF₂Cl | |
| 5-23 | Me | s-Bu | 2 | CF₂Cl | |
| 5-24 | Me | t-Bu | 2 | CF₂Cl | |
| 5-25 | Et | Me | 0 | CF₂Cl | |
| 5-26 | Et | Et | 0 | CF₂Cl | |
| 5-27 | Et | n-Pr | 0 | CF₂Cl | |
| 5-28 | Et | i-Pr | 0 | CF₂Cl | |
| 5-29 | Et | n-Bu | 0 | CF₂Cl | |
| 5-30 | Et | i-Bu | 0 | CF₂Cl | |
| 5-31 | Et | s-Bu | 0 | CF₂Cl | |
| 5-32 | Et | t-Bu | 0 | CF₂Cl | |
| 5-33 | Et | Me | 1 | CF₂Cl | |
| 5-34 | Et | Et | 1 | CF₂Cl | |
| 5-35 | Et | n-Pr | 1 | CF₂Cl | |
| 5-36 | Et | i-Pr | 1 | CF₂Cl | |
| 5-37 | Et | n-Bu | 1 | CF₂Cl | |
| 5-38 | Et | i-Bu | 1 | CF₂Cl | |
| 5-39 | Et | s-Bu | 1 | CF₂Cl | |
| 5-40 | Et | t-Bu | 1 | CF₂Cl | |
| 5-41 | Et | Me | 2 | CF₂Cl | |
| 5-42 | Et | Et | 2 | CF₂Cl | |
| 5-43 | Et | n-Pr | 2 | CF₂Cl | |
| 5-44 | Et | i-Pr | 2 | CF₂Cl | |
| 5-45 | Et | n-Bu | 2 | CF₂Cl | |
| 5-46 | Et | i-Bu | 2 | CF₂Cl | |
| 5-47 | Et | s-Bu | 2 | CF₂Cl | |
| 5-48 | Et | t-Bu | 2 | CF₂Cl | |
| 5-49 | Me | Me | 0 | CFCl₂ | |
| 5-50 | Me | Et | 0 | CFCl₂ | |
| 5-51 | Me | n-Pr | 0 | CFCl₂ | |
| 5-52 | Me | i-Pr | 0 | CFCl₂ | |
| 5-53 | Me | n-Bu | 0 | CFCl₂ | |
| 5-54 | Me | i-Bu | 0 | CFCl₂ | |
| 5-55 | Me | s-Bu | 0 | CFCl₂ | |
| 5-56 | Me | t-Bu | 0 | CFCl₂ | |
| 5-57 | Me | Me | 1 | CFCl₂ | |
| 5-58 | Me | Et | 1 | CFCl₂ | |
| 5-59 | Me | n-Pr | 1 | CFCl₂ | |
| 5-60 | Me | i-Pr | 1 | CFCl₂ | |
| 5-61 | Me | n-Bu | 1 | CFCl₂ | |
| 5-62 | Me | i-Bu | 1 | CFCl₂ | |
| 5-63 | Me | s-Bu | 1 | CFCl₂ | |
| 5-64 | Me | t-Bu | 1 | CFCl₂ | |
| 5-65 | Me | Me | 2 | CFCl₂ | |
| 5-66 | Me | Et | 2 | CFCl₂ | |
| 5-67 | Me | n-Pr | 2 | CFCl₂ | |
| 5-68 | Me | i-Pr | 2 | CFCl₂ | |
| 5-69 | Me | n-Bu | 2 | CFCl₂ | |
| 5-70 | Me | i-Bu | 2 | CFCl₂ | |
| 5-71 | Me | s-Bu | 2 | CFCl₂ | |
| 5-72 | Me | t-Bu | 2 | CFCl₂ | |
| 5-73 | Et | Me | 0 | CFCl₂ | |
| 5-74 | Et | Et | 0 | CFCl₂ | |
| 5-75 | Et | n-Pr | 0 | CFCl₂ | |
| 5-76 | Et | i-Pr | 0 | CFCl₂ | |
| 5-77 | Et | n-Bu | 0 | CFCl₂ | |
| 5-78 | Et | i-Bu | 0 | CFCl₂ | |
| 5-79 | Et | s-Bu | 0 | CFCl₂ | |
| 5-80 | Et | t-Bu | 0 | CFCl₂ | |
| 5-81 | Et | Me | 1 | CFCl₂ | |
| 5-82 | Et | Et | 1 | CFCl₂ | |
| 5-83 | Et | n-Pr | 1 | CFCl₂ | |
| 5-84 | Et | i-Pr | 1 | CFCl₂ | |
| 5-85 | Et | n-Bu | 1 | CFCl₂ | |
| 5-86 | Et | i-Bu | 1 | CFCl₂ | |
| 5-87 | Et | s-Bu | 1 | CFCl₂ | |
| 5-88 | Et | t-Bu | 1 | CFCl₂ | |
| 5-89 | Et | Me | 2 | CFCl₂ | |
| 5-90 | Et | Et | 2 | CFCl₂ | |
| 5-91 | Et | n-Pr | 2 | CFCl₂ | |
| 5-92 | Et | i-Pr | 2 | CFCl₂ | |
| 5-93 | Et | n-Bu | 2 | CFCl₂ | |
| 5-94 | Et | i-Bu | 2 | CFCl₂ | |
| 5-95 | Et | s-Bu | 2 | CFCl₂ | |
| 5-96 | Et | t-Bu | 2 | CFCl₂ | |
| 5-97 | Me | Me | 0 | CCl₃ | |
| 5-98 | Me | Et | 0 | CCl₃ | |
| 5-99 | Me | n-Pr | 0 | CCl₃ | |
| 5-100 | Me | i-Pr | 0 | CCl₃ | |
| 5-101 | Me | n-Bu | 0 | CCl₃ | |
| 5-102 | Me | i-Bu | 0 | CCl₃ | |
| 5-103 | Me | s-Bu | 0 | CCl₃ | |
| 5-104 | Me | t-Bu | 0 | CCl₃ | |
| 5-105 | Me | Me | 1 | CCl₃ | |
| 5-106 | Me | Et | 1 | CCl₃ | |
| 5-107 | Me | n-Pr | 1 | CCl₃ | |
| 5-108 | Me | i-Pr | 1 | CCl₃ | |
| 5-109 | Me | n-Bu | 1 | CCl₃ | |
| 5-110 | Me | i-Bu | 1 | CCl₃ | |
| 5-111 | Me | s-Bu | 1 | CCl₃ | |
| 5-112 | Me | t-Bu | 1 | CCl₃ | |
| 5-113 | Me | Me | 2 | CCl₃ | |
| 5-114 | Me | Et | 2 | CCl₃ | |
| 5-115 | Me | n-Pr | 2 | CCl₃ | |
| 5-116 | Me | i-Pr | 2 | CCl₃ | |
| 5-117 | Me | n-Bu | 2 | CCl₃ | |
| 5-118 | Me | i-Bu | 2 | CCl₃ | |
| 5-119 | Me | s-Bu | 2 | CCl₃ | |
| 5-120 | Me | t-Bu | 2 | CCl₃ | |
| 5-121 | Et | Me | 0 | CCl₃ | |
| 5-122 | Et | Et | 0 | CCl₃ | |
| 5-123 | Et | n-Pr | 0 | CCl₃ | |
| 5-124 | Et | i-Pr | 0 | CCl₃ | |
| 5-125 | Et | n-Bu | 0 | CCl₃ | |
| 5-126 | Et | i-Bu | 0 | CCl₃ | |
| 5-127 | Et | s-Bu | 0 | CCl₃ | |
| 5-128 | Et | t-Bu | 0 | CCl₃ | |
| 5-129 | Et | Me | 1 | CCl₃ | |
| 5-130 | Et | Et | 1 | CCl₃ | |
| 5-131 | Et | n-Pr | 1 | CCl₃ | |
| 5-132 | Et | i-Pr | 1 | CCl₃ | |
| 5-133 | Et | n-Bu | 1 | CCl₃ | |
| 5-134 | Et | i-Bu | 1 | CCl₃ | |
| 5-135 | Et | s-Bu | 1 | CCl₃ | |
| 5-136 | Et | t-Bu | 1 | CCl₃ | |
| 5-137 | Et | Me | 2 | CCl₃ | |
| 5-138 | Et | Et | 2 | CCl₃ | |
| 5-139 | Et | n-Pr | 2 | CCl₃ | |
| 5-140 | Et | i-Pr | 2 | CCl₃ | |
| 5-141 | Et | n-Bu | 2 | CCl₃ | |
| 5-142 | Et | i-Bu | 2 | CCl₃ | |
| 5-143 | Et | s-Bu | 2 | CCl₃ | |
| 5-144 | Et | t-Bu | 2 | CCl₃ | |
| 5-145 | Me | Me | 0 | C₂F₅ | |
| 5-146 | Me | Et | 0 | C₂F₅ | |
| 5-147 | Me | n-Pr | 0 | C₂F₅ | |
| 5-148 | Me | i-Pr | 0 | C₂F₅ | |
| 5-149 | Me | n-Bu | 0 | C₂F₅ | |
| 5-150 | Me | i-Bu | 0 | C₂F₅ | |
| 5-151 | Me | s-Bu | 0 | C₂F₅ | |
| 5-152 | Me | t-Bu | 0 | C₂F₅ | |
| 5-153 | Me | Me | 1 | C₂F₅ | |
| 5-154 | Me | Et | 1 | C₂F₅ | |
| 5-155 | Me | n-Pr | 1 | C₂F₅ | |
| 5-156 | Me | i-Pr | 1 | C₂F₅ | |
| 5-157 | Me | n-Bu | 1 | C₂F₅ | |
| 5-158 | Me | i-Bu | 1 | C₂F₅ | |
| 5-159 | Me | s-Bu | 1 | C₂F₅ | |
| 5-160 | Me | t-Bu | 1 | C₂F₅ | |
| 5-161 | Me | Me | 2 | C₂F₅ | |
| 5-162 | Me | Et | 2 | C₂F₅ | |
| 5-163 | Me | n-Pr | 2 | C₂F₅ | |
| 5-164 | Me | i-Pr | 2 | C₂F₅ | |
| 5-165 | Me | n-Bu | 2 | C₂F₅ | |
| 5-166 | Me | i-Bu | 2 | C₂F₅ | |
| 5-167 | Me | s-Bu | 2 | C₂F₅ | |
| 5-168 | Me | t-Bu | 2 | C₂F₅ | |
| 5-169 | Et | Me | 0 | C₂F₅ | |
| 5-170 | Et | Et | 0 | C₂F₅ | |
| 5-171 | Et | n-Pr | 0 | C₂F₅ | |
| 5-172 | Et | i-Pr | 0 | C₂F₅ | |
| 5-173 | Et | n-Bu | 0 | C₂F₅ | |
| 5-174 | Et | i-Bu | 0 | C₂F₅ | |
| 5-175 | Et | s-Bu | 0 | C₂F₅ | |
| 5-176 | Et | t-Bu | 0 | C₂F₅ | |
| 5-177 | Et | Me | 1 | C₂F₅ | |
| 5-178 | Et | Et | 1 | C₂F₅ | |
| 5-179 | Et | n-Pr | 1 | C₂F₅ | |
| 5-180 | Et | i-Pr | 1 | C₂F₅ | |
| 5-181 | Et | n-Bu | 1 | C₂F₅ | |
| 5-182 | Et | i-Bu | 1 | C₂F₅ | |
| 5-183 | Et | s-Bu | 1 | C₂F₅ | |
| 5-184 | Et | t-Bu | 1 | C₂F₅ | |
| 5-185 | Et | Me | 2 | C₂F₅ | |
| 5-186 | Et | Et | 2 | C₂F₅ | |
| 5-187 | Et | n-Pr | 2 | C₂F₅ | |
| 5-188 | Et | i-Pr | 2 | C₂F₅ | |
| 5-189 | Et | n-Bu | 2 | C₂F₅ | |
| 5-190 | Et | i-Bu | 2 | C₂F₅ | |
| 5-191 | Et | s-Bu | 2 | C₂F₅ | |
| 5-192 | Et | t-Bu | 2 | C₂F₅ | |
| 5-193 | Me | Me | 0 | CF(CF₃)₂ | |
| 5-194 | Me | Et | 0 | CF(CF₃)₂ | |
| 5-195 | Me | n-Pr | 0 | CF(CF₃)₂ | |
| 5-196 | Me | i-Pr | 0 | CF(CF₃)₂ | |
| 5-197 | Me | n-Bu | 0 | CF(CF₃)₂ | |
| 5-198 | Me | i-Bu | 0 | CF(CF₃)₂ | |
| 5-199 | Me | s-Bu | 0 | CF(CF₃)₂ | |
| 5-200 | Me | t-Bu | 0 | CF(CF₃)₂ | |
| 5-201 | Me | Me | 1 | CF(CF₃)₂ | |
| 5-202 | Me | Et | 1 | CF(CF₃)₂ | |
| 5-203 | Me | n-Pr | 1 | CF(CF₃)₂ | |
| 5-204 | Me | i-Pr | 1 | CF(CF₃)₂ | |
| 5-205 | Me | n-Bu | 1 | CF(CF₃)₂ | |
| 5-206 | Me | i-Bu | 1 | CF(CF₃)₂ | |
| 5-207 | Me | s-Bu | 1 | CF(CF₃)₂ | |
| 5-208 | Me | t-Bu | 1 | CF(CF₃)₂ | |
| 5-209 | Me | Me | 2 | CF(CF₃)₂ | |
| 5-210 | Me | Et | 2 | CF(CF₃)₂ | |
| 5-211 | Me | n-Pr | 2 | CF(CF₃)₂ | |
| 5-212 | Me | i-Pr | 2 | CF(CF₃)₂ | |
| 5-213 | Me | n-Bu | 2 | CF(CF₃)₂ | |
| 5-214 | Me | i-Bu | 2 | CF(CF₃)₂ | |
| 5-215 | Me | s-Bu | 2 | CF(CF₃)₂ | |
| 5-216 | Me | t-Bu | 2 | CF(CF₃)₂ | |
| 5-217 | Et | Me | 0 | CF(CF₃)₂ | |
| 5-218 | Et | Et | 0 | CF(CF₃)₂ | |
| 5-219 | Et | n-Pr | 0 | CF(CF₃)₂ | |
| 5-220 | Et | i-Pr | 0 | CF(CF₃)₂ | |
| 5-221 | Et | n-Bu | 0 | CF(CF₃)₂ | |
| 5-222 | Et | i-Bu | 0 | CF(CF₃)₂ | |
| 5-223 | Et | s-Bu | 0 | CF(CF₃)₂ | |
| 5-224 | Et | t-Bu | 0 | CF(CF₃)₂ | |
| 5-225 | Et | Me | 1 | CF(CF₃)₂ | |
| 5-226 | Et | Et | 1 | CF(CF₃)₂ | |
| 5-227 | Et | n-Pr | 1 | CF(CF₃)₂ | |
| 5-228 | Et | i-Pr | 1 | CF(CF₃)₂ | |
| 5-229 | Et | n-Bu | 1 | CF(CF₃)₂ | |
| 5-230 | Et | i-Bu | 1 | CF(CF₃)₂ | |
| 5-231 | Et | s-Bu | 1 | CF(CF₃)₂ | |
| 5-232 | Et | t-Bu | 1 | CF(CF₃)₂ | |
| 5-233 | Et | Me | 2 | CF(CF₃)₂ | |
| 5-234 | Et | Et | 2 | CF(CF₃)₂ | |
| 5-235 | Et | n-Pr | 2 | CF(CF₃)₂ | |
| 5-236 | Et | i-Pr | 2 | CF(CF₃)₂ | |
| 5-237 | Et | n-Bu | 2 | CF(CF₃)₂ | |
| 5-238 | Et | i-Bu | 2 | CF(CF₃)₂ | |
| 5-239 | Et | s-Bu | 2 | CF(CF₃)₂ | |
| 5-240 | Et | t-Bu | 2 | CF(CF₃)₂ | |
| 5-241 | Me | Me | 0 | CHF₂ | |
| 5-242 | Me | Et | 0 | CHF₂ | |
| 5-243 | Me | n-Pr | 0 | CHF₂ | |
| 5-244 | Me | i-Pr | 0 | CHF₂ | |
| 5-245 | Me | n-Bu | 0 | CHF₂ | |
| 5-246 | Me | i-Bu | 0 | CHF₂ | |
| 5-247 | Me | s-Bu | 0 | CHF₂ | |
| 5-248 | Me | t-Bu | 0 | CHF₂ | |
| 5-249 | Me | Me | 1 | CHF₂ | |
| 5-250 | Me | Et | 1 | CHF₂ | |
| 5-251 | Me | n-Pr | 1 | CHF₂ | |
| 5-252 | Me | i-Pr | 1 | CHF₂ | |
| 5-253 | Me | n-Bu | 1 | CHF₂ | |
| 5-254 | Me | i-Bu | 1 | CHF₂ | |
| 5-255 | Me | s-Bu | 1 | CHF₂ | |
| 5-256 | Me | t-Bu | 1 | CHF₂ | |
| 5-257 | Me | Me | 2 | CHF₂ | |
| 5-258 | Me | Et | 2 | CHF₂ | |
| 5-259 | Me | n-Pr | 2 | CHF₂ | |
| 5-260 | Me | i-Pr | 2 | CHF₂ | |
| 5-261 | Me | n-Bu | 2 | CHF₂ | |
| 5-262 | Me | i-Bu | 2 | CHF₂ | |
| 5-263 | Me | s-Bu | 2 | CHF₂ | |
| 5-264 | Me | t-Bu | 2 | CHF₂ | |
| 5-265 | Et | Me | 0 | CHF₂ | |
| 5-266 | Et | Et | 0 | CHF₂ | |
| 5-267 | Et | n-Pr | 0 | CHF₂ | |
| 5-268 | Et | i-Pr | 0 | CHF₂ | |
| 5-269 | Et | n-Bu | 0 | CHF₂ | |
| 5-270 | Et | i-Bu | 0 | CHF₂ | |
| 5-271 | Et | s-Bu | 0 | CHF₂ | |
| 5-272 | Et | t-Bu | 0 | CHF₂ | |
| 5-273 | Et | Me | 1 | CHF₂ | |
| 5-274 | Et | Et | 1 | CHF₂ | |
| 5-275 | Et | n-Pr | 1 | CHF₂ | |
| 5-276 | Et | i-Pr | 1 | CHF₂ | |
| 5-277 | Et | n-Bu | 1 | CHF₂ | |
| 5-278 | Et | i-Bu | 1 | CHF₂ | |
| 5-279 | Et | s-Bu | 1 | CHF₂ | |
| 5-280 | Et | t-Bu | 1 | CHF₂ | |
| 5-281 | Et | Me | 2 | CHF₂ | |
| 5-282 | Et | Et | 2 | CHF₂ | |
| 5-283 | Et | n-Pr | 2 | CHF₂ | |
| 5-284 | Et | i-Pr | 2 | CHF₂ | |
| 5-285 | Et | n-Bu | 2 | CHF₂ | |
| 5-286 | Et | i-Bu | 2 | CHF₂ | |
| 5-287 | Et | s-Bu | 2 | CHF₂ | |
| 5-288 | Et | t-Bu | 2 | CHF₂ | |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | |
|---|---|
| 75 Gew.-Teile einer | Verbindung der allgemeinen Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | |
|---|---|
| 25 Gew.-Teile einer | Verbindung der allgemeinen Formel (I), |
| 5 " | 2,2'-dinaphthylmethan-6,6'-disulfonsaures Natrium, |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen beziehungsweise Rhizomstücke Mono- und dikotyler Schadpflanzen werden in Töpfen von 9 bis 13 cm Durchmesser in sandiger Lehmerde ausgelegt und mit Erde bedeckt. Die als emulgierbare Konzentrate oder Stäubemittel formulierten Herbizide werden in Form wäßriger Dispersionen oder Suspensionen bzw. Emulsionen mit einer Wasseraufwandmenge von umgerechnet 300 bis 800l/ha auf die Oberfläche der Abdeckerde appliziert. Anschließend werden die Töpfe zur weiteren Kultivierung der Pflanzen im Gewächshaus unter optimalen Bedingungen gehalten. Nach 3 bis 4 Wochen Standzeit der Versuchspflanzen im Gewächshaus unter optimalen Wachstumsbedingungen wird die Wirkung der erfindungsgemäßen Verbindungen im Vergleich zu Verbindungen, die im Stand der Technik offenbart sind, bonitiert. Wie die Ergebnisse der Vergleichstabellen zeigen, weisen die ausgewählten erfindungsgemäßen Verbindungen dabei eine besssere herbizide Wirksamkeit gegen ein breites Spektrum wirtschaftlich wichtiger mono- und dikotyler Schadpflanzen auf als die im Stand der Technik offenbarten.

Die in folgenden Vergleichstabellen verwendeten Abkürzungen bedeuten: ALOMY Alopecurus myosuroides AMARE Amaranthus retroflexus AVEFA Avena fatua ECHCG Echinochloa crus galli MATIN Matricaria inodora PHBPU Pharbitis purpureum SETVI Setaria viridis VIOTR Viola tricolor

**Vergleichstabelle 1: Vorauflauf**

| Verbindung | Dosierung | herbizide Wirkung gegen | |
|---|---|---|---|
| | [g a.i./ha] | PHBPU | VIOTR |
| 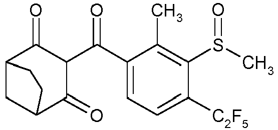 | 320 | 90% | 100% |
| erfindungsgemäße Verbindung Nr. 1-153 | | | |
| 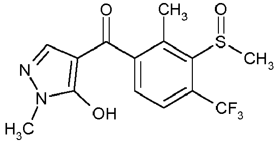 | 320 | 50% | 90% |
| aus WO 2008/125214 bekannte Verbindung Nr. 1-9 | | | |

**Vergleichstabelle 2: Vorauflauf**

| Verbindung | Dosierung | herbizide Wirkung gegen | |
|---|---|---|---|
| | [g a.i./ha] | PHBPU | VIOTR |
| 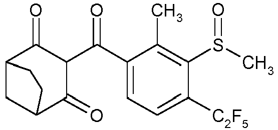 | 80 | 80% | 100% |
| erfindungsgemäße Verbindung Nr. 1-153 | | | |
| 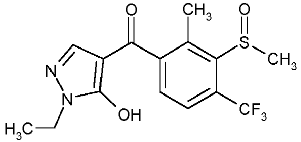 | 80 | 0% | 50% |
| aus WO 2008/125214 bekannte Verbindung Nr. 2-9 | | | |

**Vergleichstabelle 3: Vorauflauf**

| Verbindung | Dosierung | herbizide Wirkung gegen | |
|---|---|---|---|
| | [g a.i./ha] | PHBPU | VIOTR |
| 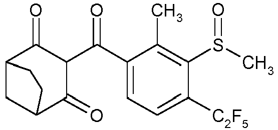 | 320 | 90% | 100% |
| erfindungsgemäße Verbindung Nr. 1-153 | | | |
| 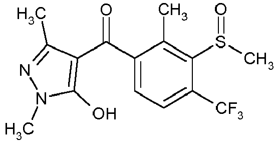 | 320 | 60% | 40% |
| aus WO 2008/125214 bekannte Verbindung Nr. 3-9 | | | |

**Vergleichstabelle 4: Vorauflauf**

| Verbindung | Dosierung | herbizide Wirkung gegen | | |
|---|---|---|---|---|
| | [g a.i./ha] | AVEFA | MATIN | PHBPU |
| 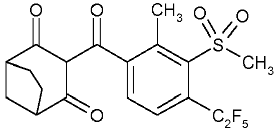 | 320 | 80% | 100% | 90% |
| erfindungsgemäße Verbindung Nr. 1-161 | | | | |
| 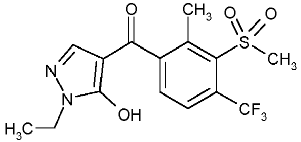 | 320 | 40% | 90% | 50% |
| aus WO 2008/125214 bekannte Verbindung Nr. 2-17 | | | | |

**Vergleichstabelle 5: Vorauflauf**

| Verbindung | Dosierung | herbizide Wirkung gegen | |
|---|---|---|---|
| | [g a.i./ha] | SETVI | VIOTR |
| 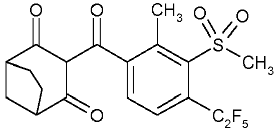 | 80 | 100% | 100% |
| erfindungsgemäße Verbindung Nr. 1-161 | | | |
| 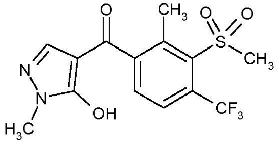 | 80 | 60% | 50% |
| aus WO 2008/125214 bekannte Verbindung Nr. 3-17 | | | |

### 2. Herbizide Wirkung gegen Schadpflanzen im Nachauflauf

Samen von mono- und dikotylen Schadpflanzen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Versuchspflanzen im Dreiblattstudium behandelt. Die als Spritzpulver bzw. als Emulsionskonzentrate formulierten erfindungsgemäßen Verbindungen werden mit einer Wasseraufwandmenge von umgerechnet 600 bis 800 l/ha auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der erfindungsgemäßen Verbindungen im Vergleich zu Verbindungen, die im Stand der Technik offenbart sind, bonitiert. Wie die Ergebnisse der Vergleichstabellen zeigen, weisen die ausgewählten erfindungsgemäßen Verbindungen dabei eine besssere herbizide Wirksamkeit gegen ein breites Spektrum wirtschaftlich wichtiger mono- und dikotyler Schadpflanzen auf als die im Stand der Technik offenbarten.

**Vergleichstabelle 6: Nachauflauf**

| Verbindung | Dosierung | herbizide Wirkung gegen | |
|---|---|---|---|
| | [g a.i./ha] | ALOMY | PHBPU |
| 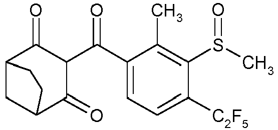 | 80 | 90% | 100% |
| erfindungsgemäße Verbindung Nr. 1-153 | | | |
| 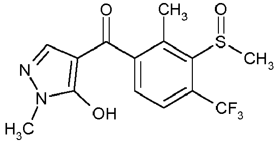 | 80 | 60% | 60% |
| aus WO 2008/125214 bekannte Verbindung Nr. 1-9 | | | |

**Vergleichstabelle 7: Nachauflauf**

| Verbindung | Dosierung | herbizide Wirkung gegen | |
|---|---|---|---|
| | [g a.i./ha] | ALOMY | PHBPU |
| 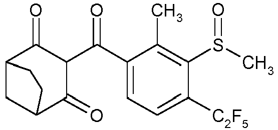 | 80 | 90% | 100% |
| erfindungsgemäße Verbindung Nr. 1-153 | | | |
| 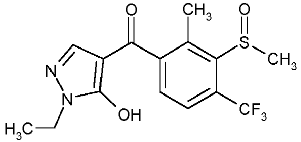 | 80 | 60% | 80% |
| aus WO 2008/125214 bekannte Verbindung Nr. 2-9 | | | |

**Vergleichstabelle 8: Nachauflauf**

| Verbindung | Dosierung | herbizide Wirkung gegen | | |
|---|---|---|---|---|
| | [g a.i./ha] | ALOMY | AMARE | PHBPU |
| 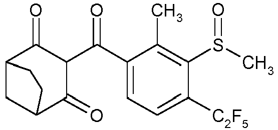 | 80 | 90% | 100% | 100% |
| erfindungsgemäße Verbindung Nr. 1-153 | | | | |
| 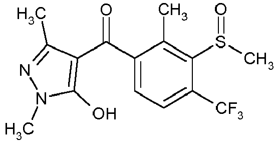 | 80 | 30% | 50% | 50% |
| aus WO 2008/125214 bekannte Verbindung Nr. 3-9 | | | | |

**Vergleichstabelle 9: Nachauflauf**

| Verbindung | Dosierung | herbizide Wirkung gegen | | |
|---|---|---|---|---|
| | [g a.i./ha] | ECHCG | MATIN | PHBPU |
| 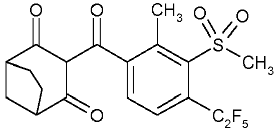 | 80 | 100% | 100% | 90% |
| erfindungsgemäße Verbindung Nr. 1-161 | | | | |
| 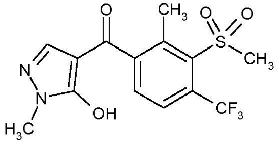 | 80 | 95% | 55% | 80% |
| aus WO 2008/125214 bekannte Verbindung Nr. 1-17 | | | | |

**Vergleichstabelle 10: Nachauflauf**

| Verbindung | Dosierung | herbizide Wirkung gegen | | |
|---|---|---|---|---|
| | [g a.i./ha] | ECHCG | MATIN | PHBPU |
| 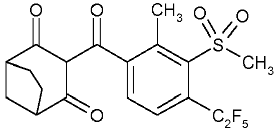 | 80 | 100% | 100% | 90% |
| erfindungsgemäße Verbindung Nr. 1-161 | | | | |
| 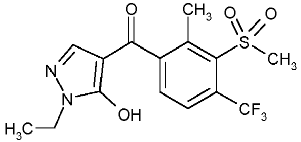 | 80 | 90% | 90% | 40% |
| aus WO 2008/125214 bekannte Verbindung Nr. 2-17 | | | | |

**Vergleichstabelle 11: Nachauflauf**

| Verbindung | Dosierung | herbizide Wirkung gegen | | |
|---|---|---|---|---|
| | [g a.i./ha] | PHBPU | SETVI | VIOTR |
| 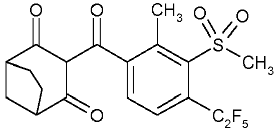 | 80 | 90% | 100% | 100% |
| erfindungsgemäße Verbindung Nr. 1-161 | | | | |
| 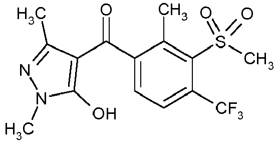 | 80 | 80% | 60% | 80% |
| aus WO 2008/125214 bekannte Verbindung Nr. 3-17 | | | | |

## Patentansprüche

1. (4-Halogenalkyl-3-thiobenzoyl)cyclohexandione der Formel (I) oder deren Salze 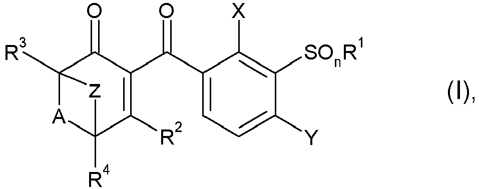 worin
X bedeutet (C₁-C₄)-Alkyl,
Y bedeutet (C₁-C₄)-Halogenalkyl mit Ausnahme von Trifluormethyl,
A und Z bedeuten unabhängig voneinander Sauerstoff, -S(O)ₘ-, -N(R⁵)-, Carbonyl oder (C₁-C₄)-Alkylen, das durch q Einheiten aus der Gruppe Sauerstoff, -S(O)ₘ-, -N(R⁵)- und Carbonyl unterbrochen ist, und das durch s Reste R⁶ substituiert ist,
R¹ bedeutet (C₁-C₄)-Alkyl,
R² bedeutet Hydroxy, SR⁷, NR⁸R⁹,
R³ und R⁴ bedeuten unabhängig voneinander Wasserstoff oder (C₁-C₄)-Alkyl,
R⁵ bedeutet Wasserstoff, (C₁-C₄)-Alkyl, (C₁-C₄)-Alkoxy, (C₁-C₄)-Alkylcarbonyl, (C₁-C₄)-Alkoxycarbonyl, Phenylcarbonyl oder Phenoxycarbonyl, wobei der Phenylring in den beiden letztgenannten Resten durch bis zu p Halogenatome und durch bis zu p Reste aus der Gruppe Nitro, Cyano, (C₁-C₄)-Alkyl, (C₁-C₄)-Halogenalkyl, (C₁-C₄)-Alkoxy und (C₁-C₄)-Halogenalkoxy substituiert ist,
R⁶ bedeutet Halogen, (C₁-C₄)-Alkyl, (C₁-C₄)-Halogenalkyl, (C₁-C₄)-Alkoxy, (C₁-C₄)-Halogenalkoxy oder (C₁-C₄)-Alkoxy-(C₁-C₄)-alkyl,
R⁷ bedeutet (C₁-C₄)-Alkyl oder durch p Halogenatome oder durch p Reste aus der Gruppe Nitro, Cyano, (C₁-C₄)-Alkyl, (C₁-C₄)-Halogenalkyl, (C₁-C₄)-Alkoxy und (C₁-C₄)-Halogenalkoxy substituiertes Phenyl,
R⁸ bedeutet Wasserstoff, (C₁-C₄)-Alkyl oder (C₁-C₄)-Alkoxy,
R⁹ bedeutet Wasserstoff oder (C₁-C₄)-Alkyl,
oder
R⁸ und R⁹ bilden mit dem Stickstoffatom, an das sie gebunden sind, einen 5- oder 6-gliedrigen gesättigten, partiell gesättigten oder vollständig ungesättigten Ring, der m weitere Heteroatome aus der Gruppe Sauerstoff, Schwefel und Stickstoff enthält, und der durch bis zu s Halogenatome und durch bis zu p Reste aus der Gruppe, Cyano, (C₁-C₄)-Alkyl, (C₁-C₄)-Halogenalkyl, (C₁-C₄)-Alkoxy und (C₁-C₄)-Halogenalkoxy substituiert ist,
m und n bedeuten unabhängig voneinander 0, 1 oder 2,
p bedeutet 0, 1, 2, 3, 4 oder 5,
q bedeutet 0 oder 1,
s bedeutet 0, 1, 2, 3, 4, 5, 6, 7 oder 8.

2. (4-Halogenalkyl-3-thiobenzoyl)cyclohexandione der Formel (I) nach Anspruch 1, worin
X bedeutet (C₁-C₄)-Alkyl,
Y bedeutet (C₁-C₄)-Halogenalkyl mit Ausnahme von Trifluormethyl,
A und Z bedeuten unabhängig voneinander Sauerstoff oder (C₁-C₄)-Alkylen,
R¹ bedeutet (C₁-C₄)-Alkyl,
R² bedeutet Hydroxy,
R³ und R⁴ bedeuten unabhängig voneinander Wasserstoff oder (C₁-C₄)-Alkyl.

3. (4-Halogenalkyl-3-thiobenzoyl)cyclohexandione der Formel (I) nach Anspruch 1, worin
X bedeutet (C₁-C₄)-Alkyl,
Y bedeutet Trichlormethyl, Difluormethyl, Dichlorfluormethyl, Chlordifluormethyl, Pentafluorethyl oder Heptafluor-iso-propyl,
A und Z bedeuten jeweils (C₁-C₄)-Alkylen,
R¹ bedeutet (C₁-C₄)-Alkyl,
R² bedeutet Hydroxy,
R³ und R⁴ bedeuten unabhängig voneinander Wasserstoff oder (C₁-C₄)-Alkyl.

4. (4-Halogenalkyl-3-thiobenzoyl)cyclohexandione nach einem der Ansprüche 1 bis 3, worin
X bedeutet Methyl,
Y Chlordifluormethyl, Difluormethyl oder Pentafluorethyl,
A und Z bedeuten unabhängig voneinander jeweils CH₂ oder CH₂CH₂,
R¹ bedeutet Methyl,
R² bedeutet Hydroxy,
R³ und R⁴ bedeuten jeweils Wasserstoff,
n bedeutet 1 oder 2.

5. (4-Halogenalkyl-3-thiobenzoyl)cyclohexandione nach einem der Ansprüche 1 bis 4, worin
X bedeutet Methyl,
Y Pentafluorethyl,
A und Z bedeuten unabhängig voneinander jeweils CH₂ oder CH₂CH₂,
R¹ bedeutet Methyl,
R² bedeutet Hydroxy,
R³ und R⁴ bedeuten jeweils Wasserstoff,
n bedeutet 1 oder 2.

6. Herbizide Mittel, **gekennzeichnet durch** einen herbizid wirksamen Gehalt an mindestens einer Verbindung der Formel (I) gemäß einem der Ansprüche 1 bis 5.

7. Herbizide Mittel nach Anspruch 6 in Mischung mit Formulierungshilfsmitteln.

8. Herbizide Mittel nach Anspruch 6 oder 7 enthaltend mindestens einen weiteren pestizid wirksame Stoffen aus der Gruppe Insektizide, Akarizide, Herbizide, Fungizide, Safenern und Wachstumsregulatoren.

9. Herbizide Mittel nach Anspruch 8 enthaltend einen Safener.

10. Herbizide Mittel nach Anspruch 8 oder 9 enthaltend ein weiteres Herbizid.

11. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 5 oder eines herbiziden Mittels nach einem der Ansprüche 6 bis 10 auf die Pflanzen oder auf den Ort des unerwünschten Pflanzenwachstums appliziert.

12. Verwendung von Verbindungen der Formel (I) gemäß einem der Ansprüche 1 bis 5 oder von herbiziden Mitteln nach einem der Ansprüche 6 bis 10 zur Bekämpfung unerwünschter Pflanzen.

13. Verwendung nach Anspruch 12, **dadurch gekennzeichnet, daß** die Verbindungen der Formel (I) zur Bekämpfung unerwünschter Pflanzen in Kulturen von Nutzpflanzen eingesetzt werden.

14. Verwendung nach Anspruch 13, **dadurch gekennzeichnet, daß** die Nutzpflanzen transgene Nutzpflanzen sind.

## Claims

1. A (4-haloalkyl-3-thiobenzoyl)cyclohexanedione of the formula (I) or salt thereof 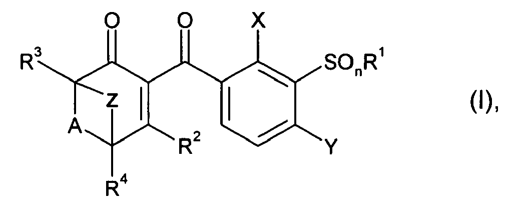 in which
X is (C₁-C₄)-alkyl,
Y is (C₁-C₉)-haloalkyl bar trifluoromethyl,
A and Z independently of one another are oxygen, -S(O)ₘ-, -N(R⁵)-, carbonyl or (C₁-C₄)-alkylene which is interrupted by q units from the group consisting of oxygen, -S(O)ₘ-, -N(R⁵)- and carbonyl, and which is substituted by s radicals R⁶,
R¹ is (C₁-C₄)-alkyl,
R² is hydroxyl, SR⁷, NR⁸R⁹,
R³ and R⁴ are independently of one another hydrogen or (C₁-C₄) -alkyl,
R⁵ is hydrogen, (C₁-C₄)-alkyl, (C₁-C₄)-alkoxy, (C₁-C₄)-alkylcarbonyl, (C₁-C₄)-alkoxycarbonyl, phenylcarbonyl or phenoxycarbonyl, the phenyl ring in the two last-mentioned radicals being substituted by up to p halogen atoms and by up to p radicals from the group consisting of nitro, cyano, (C₁-C₄)-alkyl, (C₁-C₄)-haloalkyl, (C₁-C₄)-alkoxy, and (C₁-C₄)-haloalkoxy,
R⁶ is halogen, (C₁-C₄)-alkyl, (C₁-C₄)-haloalkyl, (C₁-C₄)-alkoxy, (C₁-C₄)-haloalkoxy or (C₁-C₄)-alkoxy-(C₁-C₄)-alkyl,
R⁷ is (C₁-C₄)-alkyl or is phenyl which is substituted by p halogen atoms or by p radicals from the group consisting of nitro, cyano, (C₁-C₄)-alkyl, (C₁-C₄)-haloalkyl, (C₁-C₄)-alkoxy, and (C₁-C₄)-haloalkoxy,
R⁸ is hydrogen, (C₁-C₄)-alkyl or (C₁-C₄)-alkoxy,
R⁹ is hydrogen or (C₁-C₄)-alkyl,
or
R⁸ and R⁹, with the nitrogen atom to which they are attached, form a 5- or 6-membered saturated, partially saturated or fully unsaturated ring which contains m further heteroatoms from the group consisting of oxygen, sulfur, and nitrogen, and which is substituted by up to s halogen atoms and by up to p radicals from the group consisting of cyano, (C₁-C₄)-alkyl, (C₁-C₄)-haloalkyl, (C₁-C₄)-alkoxy, and (C₁-C₄)-haloalkoxy,
m and n independently of one another are 0, 1 or 2,
p is 0, 1, 2, 3, 4 or 5,
q is 0 or 1, and
s is 0, 1, 2, 3, 4, 5, 6, 7 or 8.

2. A (4-haloalkyl-3-thiobenzoyl)cyclohexanedione of the formula (I) as claimed in claim 1, in which
X is (C₁-C₄)-alkyl,
Y is (C₁-C₄)-haloalkyl bar trifluoromethyl,
A and Z are independently of one another oxygen or (C₁-C₄)-alkylene,
R¹ is (C₁-C₄)-alkyl,
R² is hydroxyl, and
R³ and R⁴ are independently of one another hydrogen or (C₁-C₄)-alkyl.

3. A (4-haloalkyl-3-thiobenzoyl)cyclohexanedione of the formula (I) as claimed in claim 1, in which
X is (C₁-C₄)-alkyl,
Y is trichloromethyl, difluoromethyl, dichlorofluoromethyl, chlorodifluoromethyl, pentafluoroethyl or heptafluoroisopropyl,
A and Z are each (C₁-C₄)-alkylene,
R¹ is (C₁-C₄)-alkyl,
R² is hydroxyl, and
R³ and R⁴ are independently of one another hydrogen or (C₁-C₄)-alkyl.

4. A (4-haloalkyl-3-thiobenzoyl)cyclohexanedione as claimed in any of claims 1 to 3, in which
X is methyl,
Y is chlorodifluoromethyl, difluoromethyl or pentafluoroethyl,
A and Z are independently of one another each CH₂ or CH₂CH₂,
R¹ is methyl,
R² is hydroxyl,
R³ and R⁴ are each hydrogen, and
n is 1 or 2.

5. A (4-haloalkyl-3-thiobenzoyl)cyclohexanedione as claimed in any of claims 1 to 4, in which
X is methyl,
Y is pentafluoroethyl,
A and Z are independently of one another each CH₂ or
CH₂CH₂,
R¹ is methyl,
R² is hydroxyl,
R³ and R⁴ are each hydrogen, and
n is 1 or 2.

6. A herbicidal composition which comprises a herbicidally effective amount of at least one compound of the formula (I) as claimed in any of claims 1 to 5.

7. The herbicidal composition as claimed in claim 6 in a mixture with formulation auxiliaries.

8. The herbicidal composition as claimed in claim 6 or 7, comprising at least one further pesticidally active compound from the group of insecticides, acaricides, herbicides, fungicides, safeners, and growth regulators.

9. The herbicidal composition as claimed in claim 8, comprising a safener.

10. The herbicidal composition as claimed in claim 8 or 9, comprising a further herbicide.

11. A method for controlling unwanted plants, which comprises applying an effective amount of at least one compound of the formula (I) as claimed in any of claims 1 to 5 or of a herbicidal composition as claimed in any of claims 6 to 10 to the plants or to the location of the unwanted plant growth.

12. The use of a compound of the formula (I) as claimed in any of claims 1 to 5 or of a herbicidal composition as claimed in any of claims 6 to 10 for controlling unwanted plants.

13. The use as claimed in claim 12, wherein the compound of the formula (I) is used for controlling unwanted plants in crops of useful plants.

14. The use as claimed in claim 13, wherein the useful plants are transgenic useful plants.

## Revendications

1. (4-halogénoalkyl-3-thiobenzoyl)cyclohexanediones de formule (I) ou leurs sels 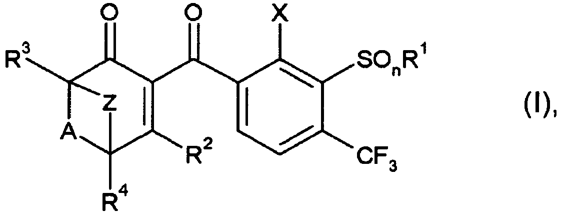 formule dans laquelle
X représente un groupe alkyle en C₁-C₄,
Y représente un groupe halogénoalkyle(C₁-C₄) à l'exclusion du groupe trifluorométhyle,
A et Z représentent indépendamment l'un de l'autre un atome d'oxygène, un groupe -S(O)ₘ-, -N(R⁵)-, carbonyle ou un groupe alkylène en C₁-C₄ qui est interrompu par q unités choisies dans le groupe constitué par un atome d'oxygène, des groupes -S(O)ₘ-, -N(R⁵)- et carbonyle, et qui est substitué par s radicaux R⁶,
R¹ représente un groupe alkyle en C₁-C₄,
R² représente un groupe hydroxy, SR⁷, NR⁸R⁹,
R³ et R⁴ représentent indépendamment l'un de l'autre un atome d'hydrogène ou un groupe alkyle en C₁-C₄,
R⁵ représente un atome d'hydrogène, un groupe alkyle en C₁-C₄, alcoxy en C₁-C₄, alkyl(C₁-C₄)carbonyle, alcoxy(C₁-C₄)carbonyle, phénylcarbonyle ou phénoxy carbonyle, le cycle phényle dans les deux radicaux nommés en dernier étant substitué par jusqu'à p atomes d'halogène et par jusqu'à p radicaux choisis dans le groupe constitué par nitro, cyano, alkyle en C₁-C₄, halogénoalkyle(C₁-C₄), alcoxy en C₁-C₄ et halogénoalcoxy(C₁-C₄),
R⁶ représente un atome d'halogène, un groupe alkyle en C₁-C₄, halogénoalkyle(C₁-C₄), alcoxy en C₁-C₄, halogénoalcoxy(C₁-C₄) ou alcoxy(C₁-C₄)-alkyle(C₁-C₄),
R⁷ représente un groupe alkyle en C₁-C₄ ou un groupe phényle substitué par p atomes d'halogène ou par p radicaux choisis dans le groupe constitué par nitro, cyano, alkyle en C₁-C₄, halogénoalkyle(C₁-C₄), alcoxy en C₁-C₄ et halogénoalcoxy(C₁-C₄),
R⁸ représente un atome d'hydrogène, un groupe alkyle en C₁-C₄ ou alcoxy en C₁-C₄,
R⁹ représente un atome d'hydrogène ou un groupe alkyle en C₁-C₄,
ou
R⁸ et R⁹ forment avec l'atome d'azote, auquel ils sont liés, un cycle à 5 ou 6 chaînons, saturé, partiellement saturé ou totalement insaturé, qui contient m autres hétéroatomes choisis dans le groupe constitué par les atomes d'oxygène, de soufre et d'azote, et est substitué par jusqu'à s atomes d'halogène et par jusqu'à p radicaux choisis dans le groupe constitué par cyano, alkyle en C₁-C₄, halogénoalkyle(C₁-C₄), alcoxy en C₁-C₄ et halogénoalcoxy(C₁-C₄),
m et n représentent indépendamment l'un de l'autre 0, 1 ou 2,
p représente 0, 1, 2, 3, 4 ou 5,
q représente 0 ou 1,
s représente 0, 1, 2, 3, 4, 5, 6, 7 ou 8.

2. (4-halogénoalkyl-3-thiobenzoyl)cyclohexanediones de formule (I) selon la revendication 1, dans lesquelles
X représente un groupe alkyle en C₁-C₄,
Y représente un groupe halogénoalkyle(C₁-C₄) à l'exclusion du groupe trifluorométhyle,
A et Z représentent indépendamment l'un de l'autre un atome d'oxygène ou un groupe alkylène en C₁-C₄,
R¹ représente un groupe alkyle en C₁-C₄,
R² représente le groupe hydroxy,
R³ et R⁴ représentent indépendamment l'un de l'autre un atome d'hydrogène ou un groupe alkyle en C₁-C₄.

3. (4-halogénoalkyl-3-thiobenzoyl)cyclohexanediones de formule (I) selon la revendication 1, dans lesquelles
X représente un groupe alkyle en C₁-C₄,
Y représente le groupe trichlorométhyle, difluorométhyle, dichlorofluorométhyle, chlorodifluorométhyle, pentafluoroéthyle ou heptafluoro-isopropyle,
A et Z représentent chacun un groupe alkylène en C₁-C₄,
R¹ représente un groupe alkyle en C₁-C₄,
R² représente le groupe hydroxy,
R³ et R⁴ représentent indépendamment l'un de l'autre un atome d'hydrogène ou un groupe alkyle en C₁-C₄.

4. (4-halogénoalkyl-3-thiobenzoyl)cyclohexanediones selon l'une quelconque des revendications 1 à 3, dans lesquelles
X représente le groupe méthyle,
Y représente le groupe chlorodifluorométhyle, difluorométhyle ou pentafluoroéthyle,
A et Z représentent chacun indépendamment l'un de l'autre CH₂ ou CH₂CH₂,
R¹ représente le groupe méthyle,
R² représente le groupe hydroxy,
R³ et R⁴ représentent chacun un atome d'hydrogène,
n représente 1 ou 2.

5. (4-halogénoalkyl-3-thiobenzoyl)cyclohexanediones selon l'une quelconque des revendications 1 à 4, dans lesquelles
X représente le groupe méthyle,
Y représente le groupe pentafluoroéthyle,
A et Z représentent chacun indépendamment l'un de l'autre CH₂ ou CH₂CH₂,
R¹ représente le groupe méthyle,
R² représente le groupe hydroxy,
R³ et R⁴ représentent chacun un atome d'hydrogène,
n représente 1 ou 2.

6. Compositions herbicides, **caractérisées par** une teneur à activité herbicide en au moins un composé de formule (I) selon l'une quelconque des revendications 1 à 5.

7. Compositions herbicides selon la revendication 6, en mélange avec des adjuvants de formulation.

8. Compositions herbicides selon la revendication 6 ou 7, contenant au moins une autre substance à activité pesticide, choisie dans le groupe constitué par les insecticides, acaricides, herbicides, fongicides, phytoprotecteurs et régulateurs de croissance.

9. Compositions herbicides selon la revendication 8, contenant un phytoprotecteur.

10. Compositions herbicides selon la revendication 8 ou 9, contenant un autre herbicide.

11. Procédé pour la lutte contre des plantes indésirables, **caractérisé en ce qu'**on applique sur les plantes ou sur le lieu de la croissance de plantes indésirables une quantité efficace d'au moins un composé de formule (I) selon l'une quelconque des revendications 1 à 5 ou d'une composition herbicide selon l'une quelconque des revendications 6 à 10.

12. Utilisation de composés de formule (I) selon l'une quelconque des revendications 1 à 5 ou de compositions herbicides selon l'une quelconque des revendications 6 à 10, pour la lutte contre des plantes indésirables.

13. Utilisation selon la revendication 12, **caractérisée en ce que** les composés de formule (I) sont utilisés pour la lutte contre des plantes indésirables dans des cultures de plantes utiles.

14. Utilisation selon la revendication 13, **caractérisée en ce que** les plantes utiles sont des plantes utiles transgéniques.
